# EUROPÄISCHE PATENTANMELDUNG

(11) **EP 4 394 384 A1**
(43) Veröffentlichungstag der Anmeldung: **03.07.2024**
(21) Anmeldenummer: 23215942.6
(22) Anmeldetag: 12.12.2023
(51) Int. Cl.: G01N 33/569, G01N 21/00

(54) **VERFAHREN ZUR DETEKTION VON ERREGERN, ANALYSEEINRICHTUNG, VERWENDUNG DER ANALYSEEINRICHTUNG UND VERWENDUNG EINES SELBSTLERNENDEN NETZWERKS ZUR AUSWERTUNG VON OPTISCHEN MESSSPEKTREN**

(30) Priorität: 27.12.2022 DE 102022134828
(71) Anmelder: Deutsches Zentrum für Luft- und Raumfahrt e.V., 53227 Bonn (DE); Trenzyme GmbH, 78467 Konstanz (DE)
(72) Erfinder: Duschek, Frank, 74239 Hardthausen (DE); Grzesiak, Jonas, 74239 Hardthausen (DE); Fellner, Lea, 74239 Hardthausen (DE); Walter, Arne, 74239 Hardthausen (DE); Horlacher, Reinhold, 78467 Konstanz (DE); Brosig, Alexander, 78467 Konstanz (DE)
(74) Vertreter: RPK Patentanwälte Reinhardt und Kaufmann Partnerschaft mbB

(57) **Zusammenfassung**

Die Erfindung betrifft ein Verfahren (1) zur Detektion von Erregern (2). Eine entnommene Testprobe (10) wird aufgereinigt, wobei der Testprobe (10) bei der Aufreinigung (S200) Mikropartikel (24) mit einer funktionalisierten Oberfläche und mit wenigstens einem selektiven Rezeptor (22) zugeführt werden, welcher den vorhandenen zu detektierenden Erreger (2) bindet. In einem Trennverfahren (S240) werden der selektive Rezeptor (22) mit den vorhanden, an den selektiven Rezeptor (22) gekoppelten zu detektierenden Erregern (2) von der Testprobe (10) getrennt. Als Messprobe (30) wird eine aufgereinigte Testprobe (20) verwendet, welche den von der Testprobe (10) getrennten selektiven Rezeptor (22) und vorhandene an den selektiven Rezeptor (22) gekoppelte Erreger (2) umfasst. In einem optischen Verfahren (S300) in Form von laserbasierter Infrarot-Spektroskopie (S320) werden die in der Messprobe (30) vorhandenen zu detektierenden Erreger (2) nachgewiesen.

Die Erfindung betrifft ferner eine Analyseeinrichtung, eine Verwendung der Analyseeinrichtung und eine Verwendung eines selbstlernenden Netzwerks.

## Beschreibung

Die Erfindung betrifft Verfahren zur Detektion eines Erregers. Des Weiteren betrifft die Erfindung eine Analyseeinrichtung, welche zur Durchführung eines Verfahrens zur Detektion von Erregern hergerichtet ist und eine Verwendung der Analyseeinrichtung. Zudem betrifft die Erfindung eine Verwendung eines selbstlernenden Netzwerks zur Auswertung von optischen Messspektren von Messproben.

### Stand der Technik

Die Corona Pandemie hat die Wichtigkeit von Testmöglichkeiten auf Infektionen mit bestimmten Erregern deutlich gemacht. Zur Detektion von Erregern und zur Überprüfung, ob ein Proband erkrankt ist, lassen sich im Wesentlichen drei Gruppen von Detektionsmethoden unterscheiden: Molekularbiologische Methoden, biochemische Methoden und Biosensoren, die eine selektive Bindung mit einem Nachweisverfahren koppeln. In der SARS-CoV2-Pandemie sind zwei Test-Technologien präsent: Der sogenannte Lateral-Flow-Schnelltest (LFT), bei dem die selektive Bindung von markierten Antikörpern zum Virus zu einem Farbsignal führt, und der PCR-Test, bei dem Teile der viralen RNA/DNA zuerst vervielfältigt und dann nachgewiesen werden. Diese Methoden sind auch auf andere Erreger anpassbar.

Aus der Fachzeitschrift Scientific Reports ist aus dem Artikel von Helinä Heino et al.: "Diagnostic performance of attenuated total reflection Fourier-transform infrared spectroscopy for detecting COVID-19 from routine nasopharyngeal swab samples", Sci. Rep. (elektronisch veröffentlicht 27.11.2022) 12:20358, Druckseiten 1-9, die Detektion von Viren mittels ATR-Infrarotspektroskopie bekannt.

### Offenbarung der Erfindung

Die Aufgabe der Erfindung ist es, ein Verfahren bereitzustellen, welches eine zuverlässige und schnelle Detektion von Erregern, insbesondere von Viren, Virengruppen oder Bakterien ermöglicht.

Eine weitere Aufgabe der Erfindung ist es, eine Analyseeinrichtung bereitzustellen, welche zur Durchführung eines Verfahrens zur Detektion von Erregern hergerichtet ist und welche eine zuverlässige und schnelle Detektion von Erregern, insbesondere von Viren, Virengruppen oder Bakterien ermöglicht.

Eine weitere Aufgabe der Erfindung ist es, eine Verwendung der Analyseeinrichtung bereitzustellen, welche eine zuverlässige und schnelle Detektion von Erregern, insbesondere von Viren, Virengruppen oder Bakterien, ermöglicht.

Eine weitere Aufgabe der Erfindung ist es, eine Verwendung eines selbstlernenden Netzwerks zur Auswertung von optischen Messspektren einer Analyseeinrichtung bereitzustellen, welches eine zuverlässige Detektion von Erregern, insbesondere von Viren, Virengruppen oder Bakterien, ermöglicht und sich zudem einfach an Veränderungen der Erreger anpasst.

Die Aufgaben werden durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Die in den Patentansprüchen einzeln aufgeführten Merkmale sind in technologisch sinnvoller Weise miteinander kombinierbar und können durch erläuternde Sachverhalte aus der Beschreibung und durch Details aus den Figuren ergänzt werden, wobei weitere Ausführungsvarianten der Erfindung aufgezeigt werden.

Im Folgenden kann auf das SARS-CoV2-Virus, auf eine Detektion des SARS-CoV2-Virus und auf Methoden zum Nachweis des SARS-CoV2-Virus hingewiesen werden. Das erfindungsgemäße Verfahren, die erfindungsgemäße Analyseeinrichtung, die erfindungsgemäße Verwendung einer Analyseeinrichtung und die erfindungsgemäße Verwendung eines selbstlernenden Netzwerks können zum Nachweis von Erregern im Allgemeinen verwendet werden und sind nicht auf den Nachweis des SARS-CoV2-Virus beschränkt. Dadurch können selbstverständlich auch andere Viren, Virengruppen oder auch Bakterien durch das erfindungsgemäße Verfahren, die erfindungsgemäße Analyseeinrichtung, die erfindungsgemäße Verwendung einer Analyseeinrichtung und die erfindungsgemäße Verwendung eines selbstlernenden Netzwerks nachgewiesen werden.

Es folgen Begriffsdefinitionen, welche für das erfindungsgemäße Verfahren, die erfindungsgemäße Analyseeinrichtung, die erfindungsgemäße Verwendung einer Analyseeinrichtung und die erfindungsgemäße Verwendung eines selbstlernenden Netzwerks gelten.

Unter einer Testprobe wird im Folgenden eine Probe eines auf den zu detektierenden Erreger zu testenden Probanden verstanden. Diese Probe kann durch einem Abstrich im Mund oder der Nase des Probanden entnommen werden. Alternativ kann die Probe eine Blutprobe, eine Urinprobe, eine Speichelprobe, eine Gewebeprobe, beispielsweise eine Hautprobe, oder jegliche andere Probe sein. Die Art der Entnahme und die Art der Testprobe können davon abhängig sein, wo der zu detektierende Erreger am besten nachgewiesen werden kann.

Die Testprobe eines Probanden, welcher den zu detektierenden Erreger im Körper aufweist, kann neben dem zu detektierenden Erreger auch andere Erreger, und/oder Verunreinigungen, und/oder Proteine und/oder Kohlehydrate und/oder die für die Entnahmeform typischen Körperflüssigkeiten, wie beispielsweise Speichel, und/oder Nasensekret, und/oder Blut und/oder Urin und/oder Wundsekret, mit den typischen Schwankungen in der Zusammensetzung aufweisen.

Die Testprobe eines Probanden, welcher den zu detektierenden Erreger nicht im Körper aufweist, kann andere Erreger und/oder Verunreinigungen und/oder Proteine und/oder Kohlenhydrate und/oder die für die Entnahmeform typischen Körperflüssigkeiten, wie beispielsweise Speichel, und/oder Nasensekret, und/oder Blut und/oder Urin und/oder Wundsekret, mit den typischen Schwankungen in der Zusammensetzung aufweisen.

Die Testprobe kann an einem Ort entnommen werden, welcher nicht dem Ort einer Detektion und/oder einer Aufreinigung und/oder eines Nachweises entspricht. Des Weiteren können mehrere Testproben gleichzeitig von mehreren Probanden an gleichen oder verschiedenen Orten entnommen und zur Detektion der zu detektierenden Erreger zum Ort der Aufreinigung der Testproben oder zum Ort der Aufreinigung der Testproben und des Nachweises des zu detektierenden Erregers transportiert werden. Hierbei können viele Testproben parallel und/oder zeitgleich oder zeitlich versetzt an einem oder an mehreren Orten entnommen werden. Des Weiteren können mehrere Testproben, ähnlich wie bei bekannten Pooltests zu einer Testprobe zusammengefügt werden, und diese Testprobe kann in weiteren Schritten aufgereinigt werden, um den Erreger nachzuweisen. Pooltests machen insbesondere bei miteinander in Verbindung stehenden Probanden Sinn, beispielsweise bei Schulklassen, Ausflugsgruppen, Arbeitsgruppen und so weiter.

Unter selektiven Rezeptoren werden im Folgenden Binder verstanden, welche jeglichen bindenden Proteinen und/oder Molekülen biologischen Ursprungs entsprechen können, welche bestimmte Erreger, beispielsweise nach einem Schlüssel-Schloss-Prinzip, an sich binden. Mögliche Beispiele für Binder sind: Antikörper, insbesondere so genannte single-chain-Antikörper, Antikörperfragmente, DNA, RNA, Nanobodys, Aptamere und/oder andere geeignete Proteine bzw. andere geeignete Proteinketten, wie beispielsweise biologische Interaktionspartner oder andere geeignete Moleküle. Die selektiven Rezeptoren können Antikörper sein, welche im Körper eines Menschen oder eines Tiers entstehen. Des Weiteren können die selektiven Rezeptoren vollsynthetisch oder teilsynthetisch entwickelt und hergestellt werden.

Hierbei kann ein selektiver Rezeptor derart gestaltet sein, dass dieser lediglich eine bestimmte Art von Erreger an sich bindet. Alternativ kann der selektive Rezeptor derart gestaltet sein, dass dieser Kreuzkorrelationen aufweist und verschiedene Arten von Erregern an sich bindet.

Zum Binden von vorhandenen SARS-Cov2-Viren in einer Testprobe können die selektiven Rezeptoren als humanes Protein ACE-2 ausgebildet sein. Hierbei kann die rezeptorbindende Domäne (RBD) des Spike-Proteins an der Virenoberfläche des SARS-Cov2-Virus selektiv nach dem Schlüssel-Schloss-Prinzip an das human Protein ACE-2 anbinden. Des Weiteren können selektive Rezeptoren auch als Antikörper, welche ein Körper gegen die SARS-Cov2-Viren bildet oder als vergleichbare Proteine ausgebildet sein. Des Weiteren sind neben dem humanen Protein ACE-2 auch andere Oberflächenproteine als selektive Rezeptoren vorstellbar. Zudem können auch andere Regionen des Spike-Proteins an die selektiven Rezeptoren gebunden werden. Das heißt, dass die Bindung der SARS-Cov2-Viren an den selektiven Rezeptor nicht auf die Bindung der rezeptorbindende Domäne (RBD) des Spike-Proteins an der Virenoberfläche angewiesen ist, da auch andere Regionen des Spike-Proteins an den entsprechenden selektiven Rezeptor anbinden können.

Auch andere Erreger können an geeignete selektive Rezeptoren anbinden.

Unter einem Trennverfahren wird im Folgenden ein Verfahren verstanden, welches die selektiven Rezeptoren mit den vorhanden an die selektiven Rezeptoren gekoppelten zu detektierenden Erreger von der Testprobe trennt. Hierbei können andere Anteile der Testprobe entsorgt bzw. verworfen werden. Zudem können weitere nicht zu detektierenden Erreger oder andere Proteine an einige der selektiven Rezeptoren gebunden sein, welche mit den vorhandenen an die selektiven Rezeptoren gekoppelten zu detektierenden Erregern von der Testprobe getrennt werden.

Unter einer aufgereinigten Testprobe wird im Folgenden eine Probe verstanden, welche von der Testprobe getrennte selektive Rezeptoren und vorhandene an die selektiven Rezeptoren gekoppelte Erreger umfassen kann.

Eine aufgereinigte Testprobe eines Probanden, welche den zu detektierenden Erreger im Körper aufweist, kann die selektiven Rezeptoren und den zu detektierenden Erreger aufweisen. Zusätzlich kann die aufgereinigte Testprobe eines Probanden, welcher den zu detektierenden Erreger im Körper aufweist, weitere Erreger oder Proteine aufweisen, welche durch die verwendeten selektiven Rezeptoren gebunden sind.

Eine aufgereinigte Testprobe eines Probanden, welcher den zu detektierenden Erreger nicht im Körper aufweist, kann die selektiven Rezeptoren aufweisen. Zusätzlich kann die aufgereinigte Testprobe eines Probanden, welcher den zu detektierenden Erreger nicht im Körper aufweist, andere Erreger oder Proteine aufweisen, welche durch die verwendeten selektiven Rezeptoren gebunden sind.

Unter einer Messprobe wird im Folgenden die aufgereinigte Testprobe verstanden. In einer alternativen Ausführung kann unter der Messprobe auch die aufgereinigte Testprobe zusammen mit einer der aufgereinigten Testprobe zugesetzten Flüssigkeit verstanden werden. Die Flüssigkeit ist für die Durchführung des erfindungsgemäßen Verfahrens, für die erfindungsgemäße Analyseeinrichtung, für die Durchführung der erfindungsgemäßen Verwendung einer Analyseeinrichtung und für die erfindungsgemäße Verwendung eines selbstlernenden Netzwerks nicht zwingend notwendig. Die Flüssigkeit kann beispielsweise reines Wasser (H₂O) sein. Ein optisches Verfahren kann leichter und effektiver mit flüssigen Testroben erfolgen. Beispielsweise fallen bei einer Transmissionsspektroskopie Streu- und Reflexionsprozesse an Oberflächen weg. Die Partikel bilden in der Flüssigkeit eine Suspension, dadurch ergibt sich eine Streuung an Partikeln, diese ist aber geringer als die Streuung an einem Feststoff. Für die Transmissionsspektroskopie wird die Testprobe beispielsweise in einer Dünnschichtzelle mit zwei Sichtfenstern angeordnet. Ein geeigneter Lichtstrahl, insbesondere im infraroten Spektralbereich, kann durch die Fenster und durch die zwischen den Fenstern angeordnete Probe geleitet werden. Die Testprobe kann auch anstatt in der Dünnschichtzelle auch in einer Kapillare oder in einer anderen geeigneten Anordnung angeordnet werden.

Ein alternatives optisches Verfahren zur Transmissionsspektroskopie ist die attenuated total reflection ATR-Infrarotspektroskopie. Hierbei können die Testproben auf dem Probenträger auch antrocknen, um die Konzentration zu erhöhen und um beispielsweise Wasserabsorption zu verringern. Die ATR-Messmethode basiert auf der Absorption des geeigneten Lichtstrahls an der Testprobe.

Unter Verunreinigungen werden im Folgenden folgende Substanzen und/oder eine Kombination der folgenden Substanzen verstanden: Proteine oder Kohlenhydrate oder nicht zu detektierende Erreger, wie beispielsweise Viren und/oder Bakterien, oder Körperflüssigkeiten, wie beispielsweise Speichel und/oder Urin und/oder Blut und/oder Gewebe und/oder Wundflüssigkeit oder andere Substanzen. Die Zusammensetzung der Verunreinigungen kann von Proband zu Proband variieren. Zudem kann die Zusammensetzung beispielsweise auch davon abhängig sein, wann und was zuletzt als Mahlzeit eingenommen wurde oder ob weitere Erkrankungen vorliegen.

Unter einem optischen Verfahren kann im Folgenden, insbesondere ein laserbasiertes Infrarot-Spektroskopieverfahren, verstanden werden. Hierbei kann ein geeigneter Infrarotstrahl auf die Messprobe gerichtet werden und ein von den Komponenten der Testprobe abhängiges Messspektrum, beispielsweise ein Absorptionsspektrum und/oder ein Transmissionsspektrum und/oder ein Reflexionsspektrum, aufgenommen werden. Hierbei können die zu detektierenden Erreger durch Eigenheiten des Messspektrums nachgewiesen werden. Zu diesem Zweck kann das aufgenommene Messspektrum mit wenigstens einem Referenzspektrum verglichen werden. Das Referenzspektrum kann einem Spektrum einer Referenzprobe mit dem zu detektierenden Erreger entsprechen. Zusätzlich oder alternativ kann das Referenzspektrum einem Spektrum einer Referenzprobe ohne dem zu detektierenden Erreger entsprechen. Hierbei können in einem möglichen Auswerteverfahren die Referenzspektren von den Messspektren abgezogen werden. Zudem sind weitere geeignete Anpassungen möglich.

Wird beispielsweise von einem Messspektrum einer Messprobe mit zu detektierenden Erregern ein Referenzspektrum einer Referenzprobe ohne zu detektierende Erreger abgezogen, können die von den zu detektierenden Erregern verursachten Peaks im Differenzspektrum deutlicher hervortreten und erkannt werden.

Wird beispielsweise von einem Messspektrum einer Messprobe ohne zu detektierende Erreger ein Referenzspektrum einer Referenzprobe ohne zu detektierende Erreger abgezogen, können sich die Peaks nahezu aufheben und das Fehlen der von zu detektierenden Erregern verursachten Peaks ebenfalls leicht erkannt werden.

Wird beispielsweise von einem Messspektrum einer Messprobe mit zu detektierenden Erregern ein Referenzspektrum einer Referenzprobe mit zu detektierenden Erregern abgezogen, können sich die Peaks nahezu aufheben und dadurch auf das vorhanden der zu detektierenden Erregern hinweisen.

Wird beispielsweise von einem Messspektrum einer Messprobe ohne zu detektierende Erreger ein Referenzspektrum einer Referenzprobe mit zu detektierenden Erregern abgezogen, können die von den zu detektierenden Erregern verursachten Peaks des Referenzspektrums im Differenzspektrum deutliche hervortreten und dadurch auf ein Fehlen der zu detektierenden Erreger in der entsprechenden Messprobe geschlossen werden.

Unter Mikropartikeln bzw. unter Beads werden im Folgenden sogenannte nano- oder mikro-Partikel mit einer funktionalisierbaren Oberfläche des Kerns verstanden. Eine spezielle Art der Mikropartikel sind magnetische Mikropartikel bzw. Magnetic Beads. Insbesondere können die magnetischen Mikropartikel ein paramagnetisches Material aufweisen, so dass magnetische Mikropartikel in ein externes Magnetfeld hineingezogen werden können.

Unter magnetischen Mikropartikeln bzw. unter Magnetic Beads werden im Folgenden sogenannte nano- oder mikro-Partikel mit einem magnetisierbaren Kern verstanden, wobei die Oberfläche des Kerns funktionalisiert werden kann.

Anstelle von magnetischen Mikropartikeln können auch nicht magnetische Mikropartikel mit einer funktionalisierbaren Oberfläche des Kerns verwendet werden. Magnetische Mikropartikel haben den Vorteil, dass diese einfach mit einem Magneten von anderen Bestandteilen der Testprobe getrennt werden können.

Die funktionalisierte Oberfläche der Mikropartikel umfasst wenigstens einen selektiven Rezeptor, so dass wenigstens ein vorhandener Erreger an der funktionalisierten Oberfläche gebunden werden kann.

Im optischen Verfahren sind die Streuwinkel und Streuintensitäten an den Mikropartikeln abhängig von der Größe der Mikropartikel und den verwendeten Wellenlängen. Ungewollte, von den Mikropartikeln abhängige Streuanteile können reduziert oder vermieden werden, indem möglichst kleine Mikropartikel verwendet werden. Die Mikropartikel können eine Größe zwischen 20 µm und 0,1 µm aufweisen. Die Größe der Mikropartikel ist hierbei abhängig von der Größe des zu binden Erregers und der Größe der Messzelle. Beispielsweise ist eine durchschnittliche Größe der Mikropartikel von 1 µm vorstellbar.

Um eine Überlagerung der Spektren der Mikropartikel mit den Spektren des zu detektierenden Erregers zu verhindern oder zu erschweren, können die Mikropartikel Materialien aufweisen, deren Spektren üblicherweise die Spektren der zu detektierenden Erreger nicht überlagern. Beispielsweise können magnetische Mikropartikel Kieselgel und einen Eisenoxidkern aufweisen. Solche Kieselgel-Eisenoxid-Mikropartikel weisen üblicherweise ein glattes Absorptionsspektrum auf und stören dadurch nicht die Auswertung der Messspektren. Es sind auch andere geeignete Materialien für die Mikropartikel vorstellbar.

Unter einem positiven Testergebnis wird im Folgenden der Nachweis der zu detektierenden Erreger in einer Probe, insbesondere in einer Messprobe, verstanden.

Unter einem negativen Testergebnis wird im Folgenden das nicht Nachweisen der zu detektierenden Erreger in einer Probe, insbesondere in einer Messprobe, verstanden.

Es wird ein Verfahren zur Detektion von Erregern insbesondere von Viren oder Virusgruppen oder Bakterien vorgeschlagen, wobei eine entnommene Testprobe aufgereinigt wird. Bei der Aufreinigung der Testprobe werden Mikropartikel mit einer funktionalisierten Oberfläche und mit wenigstens einem selektiven Rezeptor der Testprobe zugeführt. Der wenigstens eine selektive Rezeptor bindet den vorhandenen zu detektierenden Erreger. Werden mehrere selektive Rezeptoren der Testprobe zugefügt, können diese jeweils einen oder mehrere vorhandene zu detektierende Erreger binden.

In einem Trennverfahren werden der selektive Rezeptor oder die selektiven Rezeptoren mit den vorhanden, an die selektiven Rezeptoren gekoppelten zu detektierenden Erreger, von der Testprobe getrennt. Als Messprobe wird eine aufgereinigte Testprobe, welche von der Testprobe getrennte selektive Rezeptoren und vorhandene an die selektiven Rezeptoren gekoppelte Erreger umfasst, verwendet. In einem optischen Verfahren in Form einer laserbasierten Infrarot-Spektroskopie werden die in der Messprobe vorhandenen zu detektierenden Erreger nachgewiesen.

Die Aufreinigung der Testprobe kann in einer Ausführungsform in einem Verfahren ähnlich einer chromatografischen Proteinaufreinigung erfolgen, wobei die Erreger an dem wenigstens einen selektiven Rezeptor bzw. Träger fest gekoppelt sind. Beispielsweise kann die Aufreinigung eine biochemische Aufreinigung sein.

Der selektive Rezeptor oder die selektiven Rezeptoren können mit der Testprobe zusammen inkubiert werden.

Die Bindung der zu detektierenden Erreger an den wenigstens einen selektiven Rezeptor kann in vorteilhafter Weise innerhalb von Millisekunden oder innerhalb weniger Minuten erfolgen. Dadurch kann das Trennverfahren zügig durchgeführt und entsprechend viele aufgereinigte Testproben aus den Testproben gewonnen werden. Durch die Bindung zwischen den zu detektierenden Erregern und den selektiven Rezeptoren ist zudem eine zuverlässige Trennung der Erreger von der Testprobe möglich. Des Weiteren ist in vorteilhafter Weise eine Parallelisierung möglich, da vielen Testproben zeitgleich wenigstens ein selektiver Rezeptor zugesetzt werden kann und auch die Trennung der aufgereinigten Testproben von den jeweiligen Testproben zeitgleich erfolgen kann.

Nach einer vorteilhaften Ausgestaltung des Verfahrens werden bei der Aufreinigung Mikropartikel mit einer funktionalisierten Oberfläche und mit wenigstens einem selektiven Rezeptor der Testprobe zugeführt. In vorteilhafter Weise kann die funktionalisierte Oberfläche den wenigstens einen selektiven Rezeptor, welcher ein hoch affiner Bindungspartner für den zu detektierenden Erreger ist, aufweisen und dadurch die Bindung des zu detektierenden Erregers an den Mikropartikel ermöglichen.

Durch die Verwendung der Mikropartikel mit selektiven Rezeptoren können Verunreinigungen in der Messprobe in vorteilhafter Weise weitgehend reduziert werden.

Des Weiteren können Mikropartikel flexibel eingesetzt und deren Dosierung je nach Bedarf einfach angepasst werden.

Des Weiteren kann im erfindungsgemäßen Verfahren eine Differenzmessung mit einer Referenzzelle durchgeführt werden, wobei durch die Differenzmessung Beiträge durch Mikropartikel und/oder Rezeptorabsorption und/oder Absorption an einer Messzelle und/oder Absorption an einer zugeführten Flüssigkeit korrigiert werden können.

Im Gegensatz zum sogenannten Lateral-Flow-Schnelltest (LFT), bei dem die selektive Bindung von markierten Antikörpern zu einem Virus zu einem Farbsignal führt, und dadurch zu einem Nachweis des Virus führt, können die in dem erfindungsgemäßen Verfahren verwendeten selektiven Rezeptoren auch Kreuzkorrelationen aufweisen und dadurch auch andere Erreger oder Erregergruppen an sich binden. Dies würde beim bekannten Lateral-Flow-Schnelltest (LFT), zu einem falsch positiven Ergebnis führen.

Im erfindungsgemäßen Verfahren kann in vorteilhafter Weise auf das Markieren der selektiven Rezeptoren verzichtet werden, da der Nachweis der zu detektierenden Erreger nicht über die Bindung der zu detektierenden Erreger an die selektiven Rezeptoren erfolgt, sondern über ein Messspektrum im optischen Verfahren erfolgt.

Da im erfindungsgemäßen Verfahren der wenigstens eine selektive Rezeptor lediglich für eine Trennung der vorhandenen zu detektierenden Erreger von der Testprobe verwendet wird, sind die Anforderungen an die selektiven Rezeptoren in vorteilhafter Weise niedriger als bei bekannten Detektionsverfahren. Dadurch können bei der Entwicklung der selektiven Rezeptoren zu einem bestimmten Erreger auch selektive Rezeptoren mit Kreuzkorrelationen zu anderen Erregern in Betracht gezogen werden.

Des Weiteren können selektive Rezeptoren gewählt werden, deren Bindung zu dem zu detektierenden Erreger geringer ist als bei Lateral-Flow-Schnelltests. Dadurch kann die Auswahl von selektiven Rezeptoren erhöht und eine Entwicklung passender selektiver Rezeptoren erleichtert werden. Da Kreuzkorrelationen im Wesentlichen eine untergeordnete Rolle spielen, können die Kosten für die Entwicklung passender selektiver Rezeptoren und auch die Zeitspanne zum Entdecken von zu dem zu detektierenden Erreger passenden selektiven Rezeptoren in vorteilhafter Weise reduziert werden. Dadurch kann in vorteilhafter Weise beim Auftreten neuer Erreger schneller reagiert und passende selektive Rezeptoren zum Aufreinigen von Testroben bereitgestellt werden.

Durch die Verwendung des wenigstens einen selektiven Rezeptors und die Trennung des wenigstens einen selektiven Rezeptors mit den an den wenigstens einen selektiven Rezeptor gebundenen Erreger können die Verunreinigungen der Messprobe im Vergleich mit der Testprobe stark reduziert werden. Durch die reduzierten Bestandteile der Messprobe gegenüber der Testprobe können Störsignale durch Verunreinigungen im optischen Verfahren in vorteilhafter Weise reduziert und eine Auswertung der Messspektren erleichtert werden.

In vorteilhaftweise Weise weist die Messprobe hochkonzentriert nahezu alle zu detektierenden Erreger der ursprünglichen Testprobe und nur einen Bruchteil der in der Testprobe vorhandenen Verunreinigungen auf. Dadurch kann die Messprobe die zu detektierenden Erreger in einer höheren Konzentration aufweisen als die Testprobe, wodurch der Nachweis der zu detektierenden Erreger in der Messprobe leichter ist als in der Testprobe. Dadurch kann der Nachweis der zu detektierenden Erreger in der Messprobe erleichtert werden.

Durch das Auswerten der Messspektren kann in vorteilhafter Weise eine Identifizierung der zu detektierenden Erreger in Anwesenheit von Verunreinigungen, beispielsweise anderer Proteine und/oder anderer Erreger und/oder anderer Substanzen erfolgen. Eventuell können sich auch strukturell sehr ähnliche Proteine bzw. Erreger, beispielsweise Mutationen von Viren spektral für eine Unterscheidung ausreichend im Messspektrum unterscheiden.

Es sind auch noch andere geeignete Auswertungsschritte vorstellbar, um von zu detektierenden Erregern verursachte Peaks besser von anderen Peaks im erfassten Spektrum zu unterscheiden oder auch schwache von zu detektierenden Erregern verursachte Peaks erkennen zu können.

In vorteilhafter Weise werden im erfindungsgemäßen Verfahren zwei Methoden kombiniert, um schnell zu gesicherten Ergebnissen zu kommen und um Nachteile der jeweiligen Methoden auszugleichen. Hierbei ist eine erste Methode das Binden der zu detektierenden Erreger an selektive Rezeptoren, sogenannte Bindungsmoleküle oder Bindungsproteine bzw. Interaktionspartner. Eine zweite Methode ist das Aufnehmen eines charakteristischen Messspektrums durch ein optisches Verfahren und das Vergleichen mit einem Referenzspektrum, insbesondere eines Referenzspektrums des zu detektierenden Erregers.

Das Verfahren ermöglicht in vorteilhafter Weise einen hohen Durchsatz bei niedriger Nachweisgrenze und großer Genauigkeit. Durch die Aufreinigung und das Trennverfahren kann auch bei einer Testprobe, in welcher die Anzahl der zu detektierenden Erreger geringer ausfällt, dennoch ein Nachweis der zu detektierenden Erreger im optischen Verfahren möglich sein. Des Weiteren können die selektiven Rezeptoren mehrere Erreger und/oder Proteine binden, da ein Nachweis der zu detektierenden Erreger erst im optischen Verfahren erfolgt.

Bei Anwendung des erfindungsgemäßen Verfahrens zum Nachweis von SARS-Cov2-Viren kann die Empfindlichkeit in vorteilhafter Weise gegenüber Selbsttests verbessert werden.

Beispielsweise kann im Speichel einer infizierten Person die virale Belastung in den ersten Tagen der Infektion im Mittel bei etwa 10E5-10E6 PFU/ml (Plaque Forming Units pro Milliliter) liegen. Durch Aufreinigung kann die Konzentration in der Messprobe um etwa eine Größenordnung auf 10E6-10E7 PFU/ml erhöht werden.

Des Weiteren ist das Verfahren in vorteilhafter Weise preiswert und dadurch als kostengünstiger Massenschnelltest geeignet.

Des Weiteren ermöglicht das Verfahren einen zuverlässigen Nachweis der zu detektierenden Erreger. Hierbei können die Testergebnisse wenige falsch positive oder falsch negative aufweisen.

Des Weiteren kann das Verfahren schnell ausgeführt werden, da eine Bindung der zu detektierenden Erreger an die selektiven Rezeptoren zügiger erfolgen kann, als das Vermehren der Erreger in anderen bekannten Testverfahren bzw. Assays. Des Weiteren kann die Aufnahme eines Messspektrums ebenfalls zügig erfolgen. Hierbei ist eine Inkubationszeit von mehreren Stunden zum Vermehren der Erreger oder zum Vermehren von Teilen der Erreger, wie dies bei PCR-Test erforderlich ist, nicht notwendig, da eine zum Detektieren der Erreger kritische Menge an Erregern in der Messprobe bereits durch die Aufreinigung erreicht werden kann. Durch die zügige Ausführung des Verfahrens ist die Nutzung des Verfahrens als Massenschnelltest beispielsweise bei Großereignissen oder an Orten mit hohem Menschenaufkommen, wie beispielsweise an Flughäfen oder Bahnhöfen, möglich.

Das Verfahren kann in vorteilhafter Weise schnell durch die Wahl geeigneter selektiver Rezeptoren und geeigneter Referenzspektren an neuartige Erreger angepasst werden. Des Weiteren ist in vorteilhafter Weise kein aufwändiges Training für das Personal notwendig. Eine schnelle Anpassung auf neuartige Erreger ist unter anderem deshalb möglich, da die Bindung des Erregers an die selektiven Rezeptoren nicht so spezifisch wie bei Antigentests sein muss und so die sehr aufwändige Entwicklung der selektiven Rezeptoren schneller erfolgen kann und auch eine schnellere Weiterentwicklung der selektiven Rezeptoren ermöglicht werden kann.

Das Verfahren kann in vorteilhafter Weise durch Methoden des maschinellen Lernens unterstützt werden. In Kombination mit maschinellem Lernen können mit genügend Messproben Klassifizierungsalgorithmen trainiert werden, die Messproben von infizierten und nicht infizierten Probanden unterscheiden können. Auch indirekte und subtile, durch die Anwesenheit des Erregers hervorgerufene, spektrale Signaturen können durch maschinelles Lernen besser erfasst werden. Die Güte der Klassifizierung kann dabei unter anderem von der Qualität der aufgenommenen Spektren sowie der Komplexität der Aufgabe abhängen. Bezüglich Mutationen sind direkte optische Verfahren in vorteilhafter Weise unempfindlich. Wenn eine Mutation den spektralen Fingerabdruck bzw. das charakteristische Messspektrum des Moleküls ändert, so wird diese Änderung detektiert. Der Klassifizierungsalgorithmus kann um diese neuen Daten erweitert werden, um belastbare Ergebnisse zu erhalten.

Nach einer vorteilhaften Ausgestaltung des Verfahrens kann zum Gewinnen einer Messprobe eine aufgereinigte Testprobe, welche den wenigstens einen von der Testprobe getrennten selektiven Rezeptor und vorhandene an den wenigstens einen selektiven Rezeptor gekoppelte Erreger umfasst, mit einer Flüssigkeit versetzt werden. Die Flüssigkeit kann beispielsweise reines Wasser (H₂O) sein. Ein optisches Verfahren kann in vorteilhafter Weise leichter und effektiver mit flüssigen Testroben erfolgen.

Nach einer vorteilhaften Ausgestaltung des Verfahrens kann als Flüssigkeit Deuteriumoxid (D₂O) verwendet werden. Deuteriumoxid, auch als schweres Wasser bekannt, hat gegenüber normalem Wasser den Vorteil, dass eine Wasserabsorption im optischen Verfahren reduziert oder in einen weniger störenden Bereich der Messspektren verschoben werden kann. Dadurch kann eine Überlagerung der Signale der zu detektierenden Erreger durch Signale des Wassers verhindert oder erschwert werden. Des Weiteren kann Deuteriumoxid eine Eindringtiefe eines Infrarot-Strahls in die Messprobe erhöhen und dadurch die Wegstrecke durch die Messprobe erhöhen, wodurch in vorteilhafter Weise die gemessenen Signalbeiträge der Erreger erhöht werden können. Eine weitere Signalerhöhung der zu detektierenden Erreger kann durch die Reduktion des Flüssigkeitsvolumens erreicht werden.

Nach einer vorteilhaften Ausgestaltung des Verfahrens können bei der Aufreinigung magnetische Mikropartikel mit einer funktionalisierten Oberfläche und mit wenigstens einem selektiven Rezeptor der Testprobe zugeführt werden.

Nach einer vorteilhaften Ausgestaltung des Verfahrens können die magnetischen Mikropartikel durch einen Magneten fixiert oder durch einen Magneten in eine vorgegebene Richtung bewegt werden. Insbesondere können die magnetischen Mikropartikel ein paramagnetisches Material aufweisen, so dass die magnetischen Mikropartikel in ein externes Magnetfeld hineingezogen werden können.

In vorteilhafter Weise können die magnetischen Mikropartikel und dadurch die selektiven Rezeptoren mit den an die selektiven Rezeptoren gebundenen Erreger einfach durch einen Magneten fixiert oder durch einen Magneten in eine vorgegebene Richtung bewegt werden. Dadurch kann die Trennung der selektiven Rezeptoren und der vorhandenen zu detektierenden Erreger von anderen Komponenten der Testprobe einfach und schnell erfolgen. Beispielsweise kann ein Probengefäß ausgeschüttet werden, während die die magnetische Mikropartikel von dem wenigstens einen Magneten fixiert sind. Alternativ können die anderen Komponenten der Testprobe abgesaugt werden.

Nach einer vorteilhaften Ausgestaltung des Verfahrens können in einem Trennverfahren die Mikropartikel vom Rest der Testprobe getrennt werden, insbesondere kann der Rest der Testprobe verworfen werden, wobei die abgetrennten Mikropartikel die aufgereinigte Testprobe bilden.

Bei der Nutzung von nicht magnetischen Mikropartikeln können Mikropartikel und die mit den Mikropartikeln gekoppelten selektiven Rezeptoren mit den an die selektiven Rezeptoren gebundenen Erregern und/oder Proteinen durch ein Sieb oder mittels Schwerkraft oder mittels Zentrifugalkraft vom Rest der Testprobe getrennt werden.

Sind die Mikropartikel als magnetische Mikropartikel ausgeführt, können diese im Trennverfahren durch einen Magneten festgehalten werden und vom Rest der Testprobe getrennt werden, insbesondere kann der Rest der Testprobe verworfen werden.

Die festgehaltenen magnetischen Mikropartikel können die aufgereinigte Testprobe bilden. In vorteilhafter Weise können die magnetischen Mikropartikel und die mit den magnetischen Mikropartikeln gekoppelten selektiven Rezeptoren mit den an die selektiven Rezeptoren gebundenen Erregern und/oder Proteinen einfach mit einem Magneten in einer vorgegebenen und vorteilhaften Position fixiert werden und der Rest der Testprobe verworfen werden.

Zu diesem Zweck kann der Magnet beispielsweise an einer Unterseite eines Probengefäßes wirken und nicht erwünschte Bestandteile der Testprobe können über eine Öffnung an einer Oberseite des Probengefäßes entsorgt werden, beispielsweise durch Kippen des Probegefäßes oder durch Absaugen der nicht erwünschten Bestandteile.

Die Nutzung eines Magneten ermöglicht eine einfache und kostengünstige sowie eine wenig aufwändige Trennung von zu detektierenden Erregern von anderen Bestandteilen der Testprobe.

Durch die Trennung der zu detektierenden Erreger von anderen Bestandteilen der Testprobe können die selektiven Rezeptoren und die vorhandenen zu detektierenden Erreger in einer hohen Konzentration mit geringen Verunreinigungen in der aufgereinigten Testprobe vorhanden sein. Unerwünschte Bestandteile der Testprobe können in vorteilhafter Weise einfach entsorgt werden. Dadurch kann das Probenvolumen der aufgereinigten Testprobe auch geringer sein als das Probenvolumen der Testprobe. Dies ist abhängig vom Volumen der Mikropartikel und vom Volumen der verworfenen Bestandteile der Testprobe. Die Konzentration der zu detektierenden Erreger ist in vorteilhafter Weise in der aufgereinigten Testprobe deutlich größer als die Konzentration der zu detektierenden Erreger in der Testprobe. Des Weiteren ist der Anteil an zusätzlichen Erregern oder Proteinen oder anderen Verunreinigungen in der aufgereinigten Testprobe deutlich geringer als in der Testprobe. Reste von Verunreinigungen in der aufgereinigten Testprobe sind möglich, stören durch die geringen Mengen jedoch nicht im optischen Verfahren. Dadurch können Signalbeiträge der vorhandenen zu detektierenden Erreger im optischen Verfahren im Vergleich zu den Signalbeiträgen der Verunreinigungen erhöht werden.

Nach einer vorteilhaften Ausgestaltung des Verfahrens können die von der Testprobe getrennten Mikropartikel zur Bildung der Messprobe in einer Flüssigkeit suspendiert werden. Beispielsweise können die Mikropartikel mit Wasser aufgeschlämmt und/oder aufgeschwemmt werden In vorteilhafter Weise kann die Menge an Flüssigkeit so gewählt werden, dass das Volumen der Messprobe geringer ist als das Volumen der Testprobe. Insbesondere kann die Menge an Flüssigkeit so gewählt werden, dass die Erregerkonzentration im Volumen der Messprobe größer ist als die Erregerkonzentration im Volumen der Testprobe.

Nach einer vorteilhaften Ausgestaltung des Verfahrens können mehrere Testproben zeitgleich parallel chemisch oder biochemisch aufgereinigt werden, insbesondere können mehrere Testproben in einer Vorbereitungsstation und in einer Trennungsanordnung parallel bearbeitet werden.

Dadurch kann der Durchsatz an durchzuführenden Test weiter erhöht werden.

Nach einer vorteilhaften Ausgestaltung des Verfahrens kann zur Erfassung des optischen Messspektrums ein durchstimmbarer Laser, insbesondere ein Quantenkaskadenlaser, verwendet werden.

In vorteilhafter Weise sind durchstimmbare Laser im mittleren Infrarotbereich, wie beispielsweise Quantenkaskadenlaser, spektral schmalbandig, durchstimmbar und weisen eine deutlich höhere Strahlungsleistung auf. Durch die höhere Strahlungsleistung können in vorteilhafter Weise die möglichen Schichtdicken von Messzellen von 5 µm auf ca. 35 µm erhöht werden, da sich die Wegstrecke, nach der der Laserstrahl noch sinnvoll detektiert werden kann, erhöht. Quantenkaskadenlaser sind auch für den Einsatz mit ATR-Kristallen gut geeignet.

Unter attenuated total reflection Infrarotspektroskopie, kurz ATR-Infrarotspektroskopie, wird im Folgenden eine Methode verstanden, bei welcher es sich um eine Art Transmissionsmessung handelt. Hierbei wechselwirkt die evaneszente Welle. Mit der ATR-Infrarotspektroskopie kann die Oberfläche fester oder flüssiger Substanzen untersucht werden, ohne dass eine zusätzliche Probenvorbereitung nötig ist. Dabei passiert ein Infrarotstrahl einen Lichtwellenleiter, beispielsweise einen ATR-Kristall, in einer Weise, die mindestens eine Reflexion von der Oberfläche, die Kontakt zu der Probe hat, ermöglicht. Durch die Intensität des reflektierten Lichtes, die von bekannten Variablen abhängt, können Rückschlüsse über die Zusammensetzung der Probe gezogen werden.

Im direkten Vergleich zwischen Fourier-Transformations-Infrarotspektroskopiesystemen und Quantenkaskadenlasersystemen können Quantenkaskadenlasersysteme eine bis zu tausendmal schnellere Datenaufnahme bei gleichem Signal- zu Rauschverhältnis zeigen.

Nach einer vorteilhaften Ausgestaltung des Verfahrens kann wenigstens ein Referenzspektrum zeitgleich mit dem Messspektrum aufgenommen und/oder wenigstens ein Referenzspektrum aufgenommen und hinterlegt werden. In vorteilhafter Weise kann bei gleichzeitiger Aufnahme des Referenzspektrums und des Messspektrums das Referenzspektrum mit der gleichen Strahlungsquelle wie das Messspektrum aufgenommen werden. Dadurch können Schwankungen und andere Einflüsse der Strahlungsquelle und/oder anderer Komponenten einer Messanordnung auf die Spektren effektiv erkannt und bei der Auswertung beachtet werden. Des Weiteren können die Einflüsse von verwendeten Mikropartikeln, insbesondere von magnetischen Mikropartikeln, erkannt und bei der Auswertung beachtet werden. Durch die Messung eines Referenzsignals durch eine zweite Messzelle mit eluierten, unbelegten Mikropartikeln wird in einem Differenzspektrum aus Messspektrum und Referenzspektrum nur die durch die an Mikropartikeln, gebundenen Partikeln induzierte spektrale Änderung betrachtet. Ein Referenzspektrum kann auch die Laserintensität, des verwendeten Lasers, während der Aufnahme des Messspektrums sein. Durch die Ermittlung der Laserintensität während der Aufnahme des Messspektrums können durch Schwankungen des Lasers verursachte Schwankungen im Messspektrum herausgerechnet werden.

Durch die Verwendung des Referenzspektrums oder der Referenzspektren bei der Auswertung des Messspektrums wird die Komplexität der gemessenen Signale signifikant verringert und die Identifikation der zu detektierenden Erreger in vorteilhafter Weise erleichtert.

Durch Messen und Hinterlegen eines Referenzspektrums kann dieses Referenzspektrum die von den zu detektierenden Erregern erzeugten Signale aufweisen, und zur Identifikation der in der Messprobe vorhandenen Erreger beitragen. Zusätzlich oder alternativ kann wenigstens ein Referenzspektrum Signale von durch Kreuzkorrelation an die selektiven Rezeptoren gebundenen zusätzlichen Erreger aufweisen. Dadurch können die zusätzlich oder alternativ zu den zu detektierenden Erregern vorhandenen Erreger in der Messprobe identifiziert werden, wodurch eine Identifikation von in der Messprobe vorhandener unterschiedlicher Erreger weiter zu vereinfacht werden kann.

Nach einer vorteilhaften Ausgestaltung des Verfahrens können bei Entdeckung neuer Erregermutationen die selektiven Rezeptoren angepasst werden. Dadurch können auch neue Erregermutationen, welche beispielsweise eine weniger starke Bindung zu den für die ursprünglichen Erreger entwickelten selektiven Rezeptoren aufweisen, neue selektive Rezeptoren gefunden werden, an welche die Erregermutation zuverlässig gebunden und eine Trennung von der Testprobe zuverlässig ermöglicht werden kann.

Die Erfindung schlägt nach einem weiteren Aspekt eine Analyseeinrichtung vor, hergerichtet zur Durchführung eines Verfahrens zur Detektion von Erregern, insbesondere von Viren oder Virusgruppen oder Bakterien. Die Analyseeinrichtung umfasst eine Vorbereitungsstation, eine Trennungsanordnung, und eine Auswertungsstation. Die Vorbereitungsstation ist als Aufreinigungsstation ausgebildet, welche Mittel zum Einfüllen von wenigstens einem selektiven Rezeptor in eine Testprobe umfasst. Die Mittel zum Einfüllen von selektiven Rezeptoren führen Mikropartikel mit einer funktionalisierten Oberfläche und mit wenigstens einem selektiven Rezeptor der Testprobe zu. Der wenigstens eine selektive Rezeptor bindet in der Testprobe vorhandene zu detektierende Erreger. Eine Trennungsanordnung umfasst Mittel, welche den wenigstens einen selektiven Rezeptor mit den an den wenigstens einen selektiven Rezeptor gekoppelten Erreger von der Testprobe trennt und dadurch eine aufgereinigte Testprobe und aus der aufgereinigte Testprobe eine Messprobe gewinnt.

Eine Auswertungsstation ist als optische Auswertungsstation ausgebildet. Die als eine laserbasierte Infrarot-Spektroskopieanordnung ausgebildete optische Auswertungsstation nimmt wenigstens ein optisches Messspektrum der Messprobe auf und vergleicht das Messspektrum der Messprobe mit wenigstens einem Referenzspektrum.

Die Aufreinigung der Testprobe kann in einer Analyseeinrichtung ähnlich einer chromatografischen Proteinaufreinigung erfolgen, wobei die Erreger an einem Träger bzw. den selektiven Rezeptor fest gekoppelt sind.

Der der wenigstens eine selektive Rezeptor kann mit der Testprobe zusammen inkubiert werden.

Durch die Verwendung der Mikropartikel mit selektiven Rezeptoren, welche hoch affine Bindungspartner für den zu detektierenden Erreger sind, können Verunreinigungen in der Messprobe in vorteilhafter Weise weitgehend reduziert werden. Die selektiven Rezeptoren können Bindungsmoleküle oder Bindungsproteine sein.

Die Auswertungsstation kann wenigstens eine Messzelle umfassen, in welcher die Testprobe oder eine Referenzprobe angeordnet ist. Diese Messzelle kann zum einen als Dünnschichtzelle zur Transmission und zum andern als abgeschwächte Total Reflexion Dünnschichtzelle ausgelegt sein. Die Messzelle kann Zinkselenid-Fenster aufweisen. Die Testprobe kann anstatt in der Dünnschichtzelle auch in einer Kapillare oder in einer anderen geeigneten Anordnung angeordnet werden. Alternativ kann eine Kapillare die Messzelle ausbilden.

Die Auswertungsstation kann eine variable Messstrecke und/oder eine variable Temperierung aufweisen. Die Messzelle kann mit der Messprobe bestückt und anschließend in die Auswertungsstation eingegeben werden. Zusätzlich oder alternativ besteht die Möglichkeit eine in der Auswertungsstation eingebaute Messzelle fluidisch mit einer Messprobe außerhalb der Auswertungsstation zu koppeln und dadurch die Messprobe über diese Kopplung in die Messzelle und in die Auswertungsstation einströmen zulassen. Hierbei kann eine Reinigung der Messzelle nach der Messung einer Messproben vorgesehen sein.

Die Auswertungsstation kann einen Laser oder eine andere geeignete Lichtquelle bzw. Infrarotstrahlungsquelle aufweisen, welche im Verlauf einer Messung den Wellenlängenbereich mit einer Geschwindigkeit von etwa 10 µm/s abscannt. Die Dauer einer Messung kann entsprechend unter einer Sekunde liegen.

In vorteilhafter Weise ist die Analyseeinrichtung transportabel und vor Ort, beispielsweise am Ort einer Großveranstaltung, einsetzbar. Hierbei können einzelne Komponenten, wie die Vorbereitungsstation, die Trennungsanordnung, und die Auswertungsstation gemeinsam oder einzeln transportiert werden.

Des Weiteren können die einzelnen Komponenten für sich ihre Aufgabe erfüllen, ohne mit anderen Komponenten gekoppelt oder verbunden zu sein. Daher ist eine Verteilung der Komponenten der Analyseeinrichtung auf mehre Standorte möglich. Bei einer Anordnung der Komponenten an einem Standort kann der Transportweg und die Transportdauer der Testproben, der aufgereinigten Testproben, der Messproben und der Messergebnisse reduziert werden.

Die Analyseeinrichtung gibt Messergebnisse mit großer Genauigkeit aus, da zwei Methoden zum Detektieren der zu detektierenden Erreger miteinander kombiniert werden. Die optische Auswertungsstation kann durch Auswertung der Messspektren die zu detektierenden Erreger nahezu sicher nachweisen oder das Vorhandensein der zu detektierenden Erreger nahezu sicher ausschließen.

Die Vorbereitungsstation und die Trennungsanordnung können Verunreinigungen in der Testprobe aus der Testprobe entfernen und einen großen Anteil der zu detektierenden Erreger von der Testprobe in eine Messprobe überführen. Durch die vergleichsweise hohe Konzentration der vorhandenen zu detektierenden Erreger in der Messprobe im Vergleich zu der Testprobe kann die Signalstärke der zu detektierenden Erreger in der Auswertungsstation verbessert werden. Des Weiteren können Störsignale durch das Entfernen von Verunreinigungen reduziert sein.

Die Analyseeinrichtung kann in vorteilhafter Weise schnell durch die Wahl geeigneter selektiver Rezeptoren und geeigneter Referenzspektren an neuartige Erreger angepasst werden. Des Weiteren ist in vorteilhafter Weise kein aufwändiges Training für das Personal notwendig. Eine schnelle Anpassung auf neuartige Erreger ist unter anderem deshalb möglich, da die Bindung des Erregers an die selektiven Rezeptoren nicht so spezifisch, wie beispielsweise bei Antigentests, sein muss und so die sehr aufwändige Entwicklung der selektiven Rezeptoren schneller erfolgen kann und auch eine schnellere Weiterentwicklung der selektiven Rezeptoren ermöglicht werden kann.

Des Weiteren kann die Analyseeinrichtung ein Messergebnis schnell ermitteln, da eine Bindung der zu detektierenden Erreger an die selektiven Rezeptoren zügiger erfolgen kann als beispielsweise das Vermehren der Erreger in anderen bekannten Testverfahren bzw. Assays. Die Aufnahme eines Messspektrums kann ebenfalls zügig erfolgen. Hierbei ist eine Inkubationszeit von mehreren Stunden zum Vermehren der Erreger oder zum Vermehren von Teilen der Erreger, wie dies bei PCR-Test erforderlich ist, nicht notwendig, da eine zum Detektieren der Erreger kritische Menge an Erregern in der Messprobe bereits durch die Aufreinigung, insbesondere der biochemischen Aufreinigung, erreicht werden kann. Dadurch ist die Analyseeinrichtung in vorteilhafter Weise zur Verwendung in einem Schnelltestzentrum geeignet.

In vorteilhafter Weise können in der Vorbereitungsstation und in der Trennungsanordnung mehrere Testproben parallel bearbeitet werden, dadurch kann der Durchsatz an durchzuführenden Test weiter erhöht werden.

In der Auswertungsstation ist ebenfalls eine parallele Bearbeitung mehrerer Messproben, beispielsweise durch die Verwendung mehrerer Laser in der Auswertungsstation, und dadurch die Aufnahme mehrere Messspektren und gegebenenfalls die Aufnahme von mehreren Referenzspektren möglich. Da die Aufnahme der Messspektren innerhalb von Sekunden erfolgt, kann ein hoher Durchsatz auch dann ermöglicht sein, wenn die Messproben einzeln und nacheinander bzw. sequentiell in der Auswertungsstation bearbeitet werden.

Nach einer vorteilhaften Ausgestaltung der Analyseeinrichtung kann die Trennungsanordnung weitere Mittel umfassen, welche von der Testprobe getrennte selektive Rezeptoren mit den gekoppelten Erregern mit einer Flüssigkeit versetzt und dadurch eine Messprobe gewinnt. Die Flüssigkeit kann beispielsweise reines Wasser (H₂O) sein. Ein optisches Verfahren kann in vorteilhafter Weise leichter und effektiver mit flüssigen Testroben erfolgen.

Nach einer vorteilhaften Ausgestaltung der Analyseeinrichtung kann die Flüssigkeit Deuteriumoxid (D₂O) entsprechen. Deuteriumoxid, auch als schweres Wasser bekannt, hat gegenüber Wasser den Vorteil, dass eine Wasserabsorption in der Auswertungsstation reduziert oder in einen weniger störenden Bereich der Messspektren verschoben werden kann.

Dadurch kann eine Überlagerung der Signale der zu detektierenden Erreger durch Signale des Wassers verhindert oder erschwert werden. Des Weiteren kann Deuteriumoxid eine Eindringtiefe eines Infrarot-Strahls oder eines geeigneten anderen Lichtstrahls in die Messprobe erhöhen und dadurch die Wegstrecke durch die Messprobe erhöhen, wodurch in vorteilhafter Weise die gemessenen Signalbeiträge der Erreger erhöht werden können. Eine weitere Signalerhöhung der zu detektierenden Erreger kann durch die Reduktion des Flüssigkeitsvolumens erreicht werden. Das Volumen der Messprobe kann geringer sein als das Volumen der Testprobe, aus welcher die Messprobe gewonnen wurde. Insbesondere die Erregerkonzentration kann im Volumen der Messprobe größer sein als die Erregerkonzentration im Volumen der Testprobe, aus welcher die Messprobe gewonnen wurde.

Nach einer vorteilhaften Ausgestaltung der Analyseeinrichtung können die Mittel zum Einfüllen von selektiven Rezeptoren in eine Testprobe Mikropartikel mit einer funktionalisierten Oberfläche und mit wenigstens einem selektiven Rezeptor der Testprobe zuführen.

Nach einer vorteilhaften Ausgestaltung der Analyseeinrichtung kann die Trennungsanordnung Mittel umfassen, welche Mikropartikel festhält und vom Rest der Testprobe trennt, wobei die festgehalten Mikropartikel eine aufgereinigte Testprobe bilden.

Bei Verwendung von magnetischen Mikropartikeln kann die Trennungsanordnung Magneten umfassen, welche die magnetischen Mikropartikel festhält und vom Rest der Testprobe trennt, wobei die festgehalten Mikropartikel eine aufgereinigte Testprobe bilden.

In vorteilhafter Weise können die magnetischen Mikropartikel und dadurch die selektiven Rezeptoren mit den an die selektiven Rezeptoren gebundenen Erreger einfach durch einen Magneten fixiert oder durch einen Magneten in eine vorgegebene Richtung bewegt werden. Dadurch kann die Trennung der selektiven Rezeptoren und der vorhandenen zu detektierenden Erreger von anderen Komponenten der Testprobe einfach und schnell erfolgen. Beispielsweise kann ein Probengefäß ausgeschüttet werden, während die die magnetische Mikropartikel von dem wenigstens einen Magneten fixiert sind. Alternativ können die anderen Komponenten der Testprobe abgesaugt werden.

Bei der Nutzung von nicht magnetischen Mikropartikeln sind andere Trennanordnungen vorstellbar. Beispielsweise können Mikropartikel und die mit den Mikropartikeln gekoppelten selektiven Rezeptoren mit den an die selektiven Rezeptoren gebundenen Erregern und/oder Proteinen, durch ein Sieb oder mittels Schwerkraft, beispielsweise mit Hilfe von Zentrifugation, vom Rest der Testprobe getrennt werden.

Nach einer vorteilhaften Ausgestaltung der Analyseeinrichtung kann die Auswertungsstation zur Erfassung des optischen Messspektrums einen durchstimmbaren Laser, insbesondere ein Quantenkaskadenlaser, und einen Sensor umfassen.

In vorteilhafter Weise sind durchstimmbare Laser im mittleren Infrarot, wie beispielsweise Quantenkaskadenlaser in vorteilhafter Weise, spektral schmalbandig, durchstimmbar und weisen eine deutlich höhere Strahlungsleistung auf. Durch die höhere Strahlungsleistung können in vorteilhafter Weise die möglichen Schichtdicken von Messzellen von 5 µm auf ca. 35 µm erhöht werden, da sich die Wegstrecke, nach der der Laserstrahl noch sinnvoll detektiert werden kann, erhöht. Quantenkaskadenlaser sind auch für den Einsatz mit ATR-Kristallen gut geeignet.

Unter attenuated total reflection Infrarotspektroskopie, kurz ATR-Infrarotspektroskopie, wird im Folgenden eine Methode verstanden, bei welcher es sich um eine Art Transmissionsmessung handelt, hierbei wechselwirkt die evaneszente Welle. Mit der ATR-Infrarotspektroskopie kann eine Oberfläche fester oder flüssiger Substanzen untersucht werden, ohne dass eine zusätzliche Probenvorbereitung nötig ist. Dabei passiert ein Infrarotstrahl einen Lichtwellenleiter, in diesem Fall den ATR-Kristall, in einer Weise, die mindestens eine Reflexion von der Oberfläche, die Kontakt zu der Probe hat, ermöglicht. Durch die Intensität des reflektierten Lichtes, die von bekannten Variablen abhängt, können Rückschlüsse über die Zusammensetzung der Probe gezogen werden.

Im direkten Vergleich zwischen Fourier-Transformations-Infrarotspektroskopiesystemen und Quantenkaskadenlasersystemen können Quantenkaskadenlasersystemen eine bis zu tausendmal schnellere Datenaufnahme bei gleichem Signal- zu Rauschverhältnis zeigen.

Es sind Quantenkaskadenlaser mit mehreren Modulen vorstellbar, welche zusammen ein kontinuierliches Spektrum zwischen 6,7 m und 11,1 m ausgeben. Der verfügbare Wellenlängenbereich kann über ein zusätzliches Modul bis 5,8 m erweitert werden. Es kann sich um einen gepulsten Laser mit einer Wiederholrate von 100 Hz bis 3 MHz und einer mittleren Leistung von 500 mW handeln.

Die Auswertungsstation kann zusätzlich weitere Komponenten aufweisen. Beispielsweise lässt sich durch den Einsatz eines Lock-in Verstärkers in Kombination mit dem gepulsten Quantenkaskadenlaser ein besonders hohes Signal/Rausch Verhältnis realisieren.

Der Sensor empfängt die transmittierte oder reflektierte Strahlung der Messzelle, aus welchen das Messspektrum und/oder das Referenzspektrum resultieren.

Nach einer vorteilhaften Ausgestaltung der Analyseeinrichtung kann die Auswertungsstation wenigstens ein Referenzspektrum zeitgleich mit dem Messspektrum aufnehmen. Zusätzlich oder alternativ kann die Auswertungsstation wenigstens ein Referenzspektrum aufnehmen und hinterlegen In vorteilhafter Weise kann das Referenzspektrum mit der gleichen Strahlungsquelle wie das Messspektrum aufgenommen werden. Dadurch können Schwankungen und andere Einflüsse der Strahlungsquelle und/oder anderer Komponenten der Auswertungsstation auf die Spektren effektiv erkannt und bei der Auswertung beachtet werden. Des Weiteren können die Einflüsse von verwendeten magnetischen Mikropartikeln erkannt und bei der Auswertung beachtet werden. Durch die Messung eines Referenzsignals durch eine zweite Messzelle mit eluierten, unbelegten Mikropartikeln, kann in einem Differenzspektrum aus Messspektrum und Referenzspektrum nur die durch die an Mikropartikeln gebundenen Partikel induzierte spektrale Änderung betrachtet werden. Ein Referenzspektrum kann auch die Laserintensität, des verwendeten Lasers, während der Aufnahme des Messspektrums sein. Durch die Ermittlung der Laserintensität während der Aufnahme des Messspektrums können durch Schwankungen des Lasers verursachte Schwankungen im Messspektrum herausgerechnet werden. Durch die Verwendung des Referenzspektrums oder der Referenzspektren bei der Auswertung des Messspektrums wird die Komplexität der gemessenen Signale signifikant verringert und die Identifikation der zu detektierenden Erreger in vorteilhafter Weise erleichtert.

Das aufgenommene und hinterlegte Referenzspektrum kann die von den zu detektierenden Erregern erzeugten Signale aufweisen und zur Identifikation der in der Messprobe vorhandenen Erreger beitragen. Zusätzlich oder alternativ kann wenigstens ein Referenzspektrum Signale von durch Kreuzkorrelation an die selektiven Rezeptoren gebundenen zusätzlichen Erreger aufweisen, um eine Identifikation der in der Messprobe zusätzlich oder alternativ zu den zu detektierenden Erregern vorhandenen Erreger weiter vereinfachen.

Nach einer vorteilhaften Ausgestaltung der Analyseeinrichtung kann die Auswertungsstation wenigstens eine Auswerte- und Steuereinrichtung umfassen, welche das Messspektrum auswertet und insbesondere entsprechende Signale an eine Ausgabeeinheit ausgibt.

Eine Ausgabeeinheit kann ein Drucker sein, welcher Testergebnisse ausdruckt. Alternativ sind auch andere optische und akustische Einheiten vorstellbar, welche entsprechende Signale ausgeben. Beispielsweise können Testergebnisse auch an eine App ausgegeben werden.

Unter einer Auswerte- und Steuereinrichtung wird im Folgenden eine physische Baugruppe oder eine logische Gruppe von zusammenwirkenden Hardware- und/oder Softwarekomponenten oder ein Prozessor verstanden. Die Auswerte- und Steuereinrichtung umfasst hierbei wenigstens einen Signaleingang zum Empfangen von Messspektren und/oder Referenzspektren und wenigstens einen Signalausgang zum Ausgeben von Messergebnissen. Wenigstens eine Recheneinheit kann durch eine entsprechende Programmierung die empfangen Messspektren auswerten. Insbesondere kann das Messspektrum auf Peaks in einem vorgegebenen Wellenlängenbereich, welche dem zu detektierenden Erreger zugeordnet ist, untersucht werden. Theoretisch könnte auch eine durch den Erreger verursachte spektrale Veränderung im Gesamtspektrum der Messprobe erkannt werden.

Wenigstens eine Speichereinheit kann Zwischenergebnisse und/oder Endergebnisse speichern.

Die Auswerte- und Steuereinrichtung kann als physische Baugruppe verstanden werden. Die Auswerte- und Steuereinrichtung könnte aber auch einfach auf einem Server implementiert werden. Dann ist diese Baugruppe ein Programm und nicht physisch vorhanden. Die Auswerte- und Steuereinrichtung kann Daten aus einer Cloud entnehmen oder in einer Cloud speichern.

In Kombination mit maschinellem Lernen können mit genügend Messproben Klassifizierungsalgorithmen trainiert werden, die Messproben von infizierten und nicht infizierten Probanden unterscheiden können. Auch indirekte und subtile, durch die Anwesenheit des Erregers hervorgerufene, spektrale Signaturen können durch maschinelles Lernen besser erfasst werden. Die Güte der Klassifizierung kann dabei unter anderem von der Qualität der aufgenommenen Spektren sowie der Komplexität der Aufgabe abhängen.

Die Erfindung schlägt nach einem weiteren Aspekt eine Verwendung einer Analyseeinrichtung zur Detektion von Erregern, insbesondere von Viren oder Virusgruppen vor, wobei in einer Vorbereitungsstation eine entnommene Testprobe aufgereinigt wird. Bei der Aufreinigung werden der Testprobe Mikropartikel mit einer funktionalisierten Oberfläche und mit wenigstens einem selektiven Rezeptor zugefügt, welcher den vorhandenen zu detektierenden Erreger bindet. In einer Trennungsanordnung werden in einem Trennverfahren der wenigstens eine selektive Rezeptor mit den vorhanden an den wenigstens einen selektiven Rezeptor gekoppelten zu detektierenden Erregern von der Testprobe getrennt. Als Messprobe wird eine aufgereinigte Testprobe, welche von den wenigstens einen von der Testprobe getrennten selektiven Rezeptor und vorhandene an den wenigstens einen selektiven Rezeptor gekoppelte Erreger umfasst, verwendet. In einer Auswertungsstation werden in einem optischen Verfahren, durch eine laserbasierte Infrarot-Spektroskopie, die in der Messprobe vorhandenen zu detektierenden Erreger in der Messprobe nachgewiesen.

Die Verfahrensschritte des erfindungsgemäßen Verfahrens ähneln den Verwendungsschritten der Verwendung der Analyseeinrichtung, so dass die bereits beschriebenen Verfahrensschritte mit der Verwendung kombiniert werden können.

Im Gegensatz zum sogenannten Lateral-Flow-Schnelltest (LFT), bei dem die selektive Bindung von markierten Antikörpern zu einem Virus zu einem Farbsignal führt, und dadurch zu einem Nachweis des Virus führt, können die in der Anwendung verwendeten selektiven Rezeptoren auch Kreuzkorrelationen aufweisen und dadurch auch andere Erreger oder Erregergruppen an sich binden. Dies würde beim bekannten Lateral-Flow-Schnelltest (LFT) zu einem falsch positiven Ergebnis führen.

In der erfindungsgemäßen Verwendung einer Analyseeinrichtung kann in vorteilhafter Weise auf das Markieren der selektiven Rezeptoren verzichtet werden, da der Nachweis der zu detektierenden Erreger nicht über die Bindung der detektierenden Erreger an die selektiven Rezeptoren erfolgt, sondern über ein Messspektrum im optischen Verfahren.

Da in der erfindungsgemäßen Verwendung der Analyseeinrichtung die selektiven Rezeptoren lediglich für eine Trennung der vorhandenen zu detektierenden Erreger von der Testprobe verwendet werden, sind die Anforderungen an die selektiven Rezeptoren in vorteilhafter Weise niedriger als bei bekannten Detektionsverfahren, dadurch können bei der Entwicklung der selektiven Rezeptoren zu einem bestimmten Erreger auch selektive Rezeptoren mit Kreuzkorrelationen zu anderen Erregern in Betracht gezogen werden. Des Weiteren können selektive Rezeptoren gewählt werden, deren Bindung zu dem zu detektierenden Erreger geringer ist als bei Lateral-Flow-Schnelltests. Dadurch kann die Auswahl von selektiven Rezeptoren erhöht und eine Entwicklung passender selektiver Rezeptoren erleichtert werden. Da Kreuzkorrelationen im Wesentlichen eine untergeordnete Rolle spielen, können die Kosten für die Entwicklung passender selektiver Rezeptoren und auch die Zeitspanne zum Entdecken von zu dem zu detektierenden Erreger passenden selektiven Rezeptoren in vorteilhafter Weise reduziert werden. Dadurch kann in vorteilhafter Weise beim Auftreten neuer Erreger schneller reagiert und passende selektive Rezeptoren zum Aufreinigen von Testroben bereitgestellt werden.

In vorteilhafter Weise weist die Messprobe hochkonzentriert nahezu alle zu detektierenden Erreger der ursprünglichen Testprobe und nur einem Bruchteil der in der Testprobe vorhandenen Verunreinigungen auf. Dadurch kann die Messprobe die zu detektierenden Erreger in einer höheren Konzentration aufweisen als die Testprobe, wodurch der Nachweis der zu detektierenden Erreger in der Messprobe leichter ist als in der Testprobe. Dadurch kann der Nachweis der zu detektierenden Erreger in der Messprobe erleichtert werden.

In vorteilhafter Weise werden in der erfindungsgemäßen Verwendung einer Analyseeinrichtung zwei Methoden kombiniert, um schnell zu gesicherten Ergebnissen zu kommen und um Nachteile der jeweiligen Methoden auszugleichen. Hierbei ist eine erste Methode das Binden der zu detektierenden Erreger an selektive Rezeptoren, sogenannte Bindungsmoleküle oder Bindungsproteine. Eine zweite Methode ist das Aufnehmen eines charakteristischen Messspektrums durch ein optisches Verfahren und das Vergleichen mit einem Referenzspektrum, insbesondere eines Referenzspektrums des zu detektierenden Erregers.

Die Verwendung einer Analyseeinrichtung ermöglicht in vorteilhafter Weise einen hohen Durchsatz bei niedriger Nachweisgrenze und großer Genauigkeit. Durch die Aufreinigung und das Trennverfahren kann auch bei einer Testprobe, in welcher die Anzahl der zu detektierenden Erreger geringer ausfällt, dennoch ein Nachweis der zu detektierenden Erreger im optischen Verfahren möglich sein. Des Weiteren können die selektiven Rezeptoren mehrere Erreger und/oder Proteine binden, da ein Nachweis der zu detektierenden Erreger erst im optischen Verfahren erfolgt.

Des Weiteren ermöglicht die Verwendung einer Analyseeinrichtung einen zuverlässigen Nachweis der zu detektierenden Erreger. Hierbei können die Testergebnisse wenige falsch positive oder falsch negative Testergebnisse aufweisen.

Des Weiteren kann die Verwendung einer Analyseeinrichtung schnell ausgeführt werden, da eine Bindung der zu detektierenden Erreger an die selektiven Rezeptoren und die Aufnahme eines Messspektrums schneller erfolgen kann als eine Inkubationszeit von mehreren Stunden zum Vermehren der Erreger oder zum Vermehren von Teilen der Erreger, wie dies bei PCR-Test erforderlich ist.

Dies ist möglich, da eine zum Detektieren der zu detektierenden Erreger kritische Menge an zu detektierenden Erregern in der Messprobe bereits durch die chemische Aufreinigung erreicht werden kann. Durch die zügige Ausführung der Verwendung einer Analyseeinrichtung, ist die Nutzung der Verwendung einer Analyseeinrichtung als Massenschnelltest beispielsweise bei Großereignissen oder an Orten mit hohem Menschenaufkommen, wie beispielsweise an Flughäfen oder Bahnhöfen, möglich.

Die Verwendung einer Analyseeinrichtung kann in vorteilhafter Weise schnell durch die Wahl geeigneter selektiver Rezeptoren und geeigneter Referenzspektren an neuartige Erreger angepasst werden. Des Weiteren ist in vorteilhafter Weise kein aufwändiges Training für das Personal notwendig. Eine schnelle Anpassung auf neuartige Erreger ist unteranderem deshalb möglich, da die Bindung des Erregers an die selektiven Rezeptoren nicht so selektiv wie bei Antigentests sein muss und so die sehr aufwändige Entwicklung der selektiven Rezeptoren schneller erfolgen kann, und auch eine schnellere Weiterentwicklung der selektiven Rezeptoren ermöglicht werden kann.

Die Verwendung einer Analyseeinrichtung kann in vorteilhafter Weise durch Methoden des maschinellen Lernens unterstützt werden. In Kombination mit maschinellem Lernen können mit genügend Messproben Klassifizierungsalgorithmen trainiert werden, die Messproben von infizierten und nicht infizierten Probanden unterscheiden können. Die Güte der Klassifizierung kann dabei unter anderem von der Qualität der aufgenommenen Spektren sowie der Komplexität der Aufgabe abhängen.

Bezüglich Mutationen sind direkte optische Verfahren in vorteilhafter Weise unempfindlich. Wenn eine Mutation den spektralen Fingerabdruck bzw. das charakteristische Messspektrum des Moleküls ändert, so wird diese Änderung detektiert. Der Klassifizierungsalgorithmus kann um diese neuen Daten erweitert werden, um belastbare Ergebnisse zu erhalten.

Nach einer vorteilhaften Ausgestaltung der Verwendung einer Analyseeinrichtung kann zum Gewinnen einer Messprobe eine aufgereinigte Testprobe, welche von der Testprobe getrennte selektive Rezeptoren und vorhandene an die selektiven Rezeptoren gekoppelte Erreger umfasst, mit einer Flüssigkeit versetzt werden.

Die Flüssigkeit kann beispielsweise reines Wasser (H₂O) sein. Ein optisches Verfahren kann in vorteilhafter Weise leichter und effektiver mit flüssigen Testroben erfolgen.

Die Erfindung schlägt nach einem weiteren Aspekt eine Verwendung eines selbstlernenden Netzwerks zur Auswertung von optischen Messspektren von Messproben vor, welche zum Nachweis von Erregern entnommen werden. Mehrere Messspektren werden mit und/oder ohne bekannte Erregerlast zur Auswertung hinterlegt und die Auswertung wird fortlaufend angepasst.

In vorteilhafter Weise können durch maschinelles Lernen mit genügend Messproben Klassifizierungsalgorithmen trainiert werden, welche die Messproben von infizierten und nicht infizierten Probanden unterscheiden können. Die Güte der Klassifizierung kann dabei unter anderem von der Qualität der aufgenommenen Spektren sowie der Komplexität der Aufgabe abhängen. Hierbei können die Erreger auch in kleinen Konzentrationen in der Messprobe nachgewiesen werden.

Nach einer vorteilhaften Ausgestaltung der Verwendung eines selbstlernenden Netzwerks können neue Mutationen des Erregers erkannt werden, insbesondere wobei bei Erkennen einer neuen Mutation des Erregers eine Warnung an eine Ausgabeeinheit ausgegeben wird.

In vorteilhafter Weise kann eine Änderung des spektralen Fingerabdrucks bzw. des charakteristische Messspektrum des Erregers festgestellt werden. Dadurch kann die Auswertung bezüglich Mutationen in vorteilhafter Weise unempfindlich sein. Der Klassifizierungsalgorithmus kann um diese neuen Daten erweitert werden, um belastbare Ergebnisse zu erhalten. Durch die Ausgabe der Warnung kann auf eine neue Erregermutation hingewiesen und entsprechende Schritte veranlasst werden.

Das erfindungsgemäße Verfahren, die erfindungsgemäße Analyseeinrichtung und die erfindungsgemäße Verwendung einer Analyseeinrichtung erfüllen in vorteilhafter Weise wichtige Eigenschaften eines Tests zum Nachweis von Erregern.

Beispielsweise weisen das erfindungsgemäße Verfahren, die erfindungsgemäße Analyseeinrichtung und die erfindungsgemäße Verwendung einer Analyseeinrichtung eine hohe Trefferquote und eine niedrige Fehlalarmrate auf. Zudem weisen das erfindungsgemäße Verfahren, die erfindungsgemäße Analyseeinrichtung und die erfindungsgemäße Verwendung einer Analyseeinrichtung eine kurze Dauer und eine niedrige Komplexität von der Probennahme bis zum Testergebnis auf. Des Weiteren ist eine Skalierbarkeit für den Einsatz in Testzentren möglich. Zudem können das erfindungsgemäße Verfahren, die erfindungsgemäße Analyseeinrichtung und die erfindungsgemäße Verwendung einer Analyseeinrichtung mit niedrigen Kosten durchgeführt bzw. verwendet werden und sind für den großflächigen Einsatz geeignet. Des Weiteren sind kurze Entwicklungszeiten möglich, um das erfindungsgemäße Verfahren, die erfindungsgemäße Analyseeinrichtung und die erfindungsgemäße Verwendung einer Analyseeinrichtung schnell für den Nachweis neuer Erreger bzw. für den Nachweis neuer Mutationen bekannter Erreger anwenden zu können.

### Zeichnung

Weitere Vorteile ergeben sich aus der folgenden Zeichnungsbeschreibung. In den Figuren sind Ausführungsbeispiele der Erfindung dargestellt. Die Figuren, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen beispielhaft:
- Fig. 1: ein schematisches Flussdiagramm eines Ausführungsbeispiels einer Aufreinigung eines Ausführungsbeispiels des erfindungsgemäßen Verfahrens zur Detektion von Erregern;
- Fig. 2: ein schematisches Flussdiagramm eines Ausführungsbeispiels eines optischen Verfahrens des erfindungsgemäßen Verfahrens zur Detektion von Erregern aus Figur 1;
- Fig. 3: eine schematische Darstellung eines Ausführungsbeispiels einer Testprobe;
- Fig. 4: eine schematische Darstellung eines Ausführungsbeispiels einer aufgereinigten Testprobe;
- Fig. 5: eine schematische Darstellung eines Ausführungsbeispiels einer Messprobe;
- Fig. 6: eine schematische Darstellung eines Ausführungsbeispiels eines magnetischen Mikropartikels mit mehreren selektiven Rezeptoren;
- Fig. 7: eine schematische Darstellung eines Ausführungsbeispiels einer erfindungsgemäßen Analyseeinrichtung zur Detektion von Erregern;
- Fig. 8: eine schematische Detailansicht eines Ausführungsbeispiels einer Auswertungsstation der erfindungsgemäßen Analyseeinrichtung aus Figur 7;
- Fig. 9: eine schematische Detailansicht eines ersten Ausführungsbeispiels einer Messzelle der Auswertungsstation aus Figur 8;
- Fig. 10: eine schematische Detailansicht eines zweiten Ausführungsbeispiels einer Messzelle der Auswertungsstation aus Figur 8; und
- Fig. 11: eine schematische Darstellung eines Messspektrums und eines Referenzspektrums.

### Ausführungsformen der Erfindung

In den Figuren sind gleichartige oder gleichwirkende Komponenten mit gleichen Bezugszeichen beziffert. Die Figuren zeigen lediglich Beispiele und sind nicht beschränkend zu verstehen.

Bevor die Erfindung im Detail beschrieben wird, ist darauf hinzuweisen, dass sie nicht auf die jeweiligen Bauteile der Vorrichtung sowie die jeweiligen Verfahrensschritte beschränkt ist, da diese Bauteile und Verfahren variieren können. Die hier verwendeten Begriffe sind lediglich dafür bestimmt, besondere Ausführungsformen zu beschreiben und werden nicht einschränkend verwendet. Wenn zudem in der Beschreibung oder in den Ansprüchen die Einzahl oder unbestimmte Artikel verwendet werden, bezieht sich dies auch auf die Mehrzahl dieser Elemente, solange nicht der Gesamtzusammenhang eindeutig etwas Anderes deutlich macht.

Im Folgenden verwendete Richtungsterminologie mit Begriffen wie "links", "rechts", "oben", "unten", "davor" "dahinter", "danach" und dergleichen dient lediglich dem besseren Verständnis der Figuren und soll in keinem Fall eine Beschränkung der Allgemeinheit darstellen. Die dargestellten Komponenten und Elemente, deren Auslegung und Verwendung können im Sinne der Überlegungen eines Fachmanns variieren und an die jeweiligen Anwendungen angepasst werden.

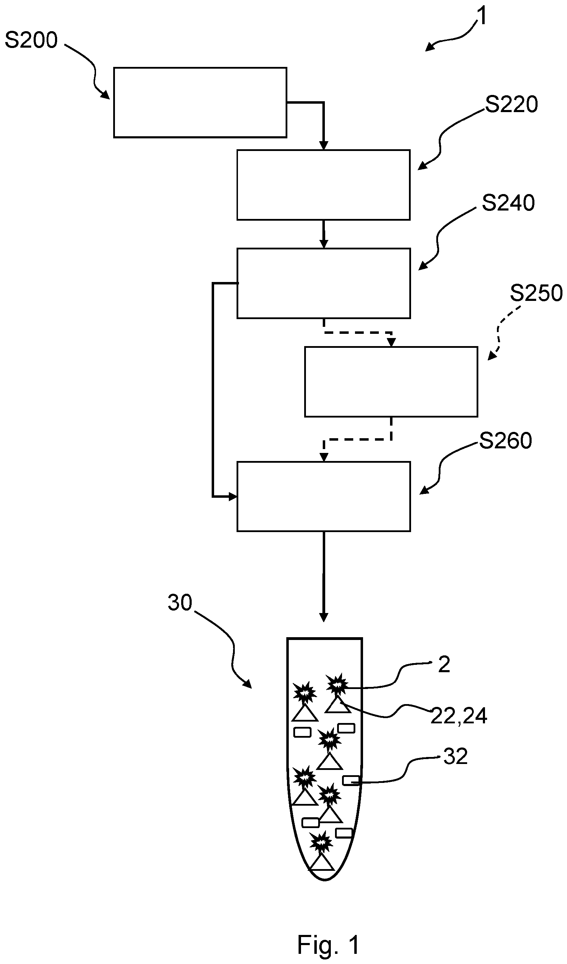

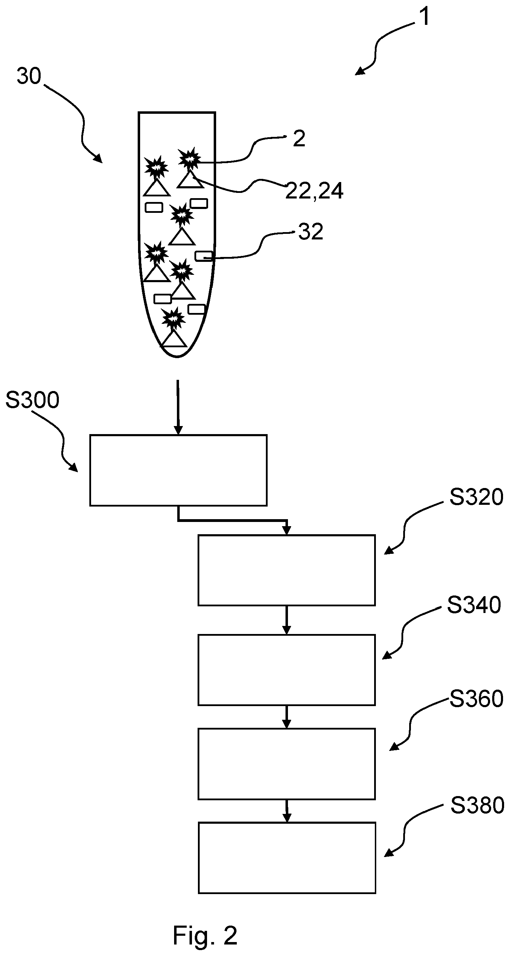

Die Figuren 1 und 2 zeigen ein Ausführungsbeispiel des erfindungsgemäßen Verfahrens 1 zur Detektion von Erregern 2, insbesondere von Viren oder Virusgruppen oder Bakterien.

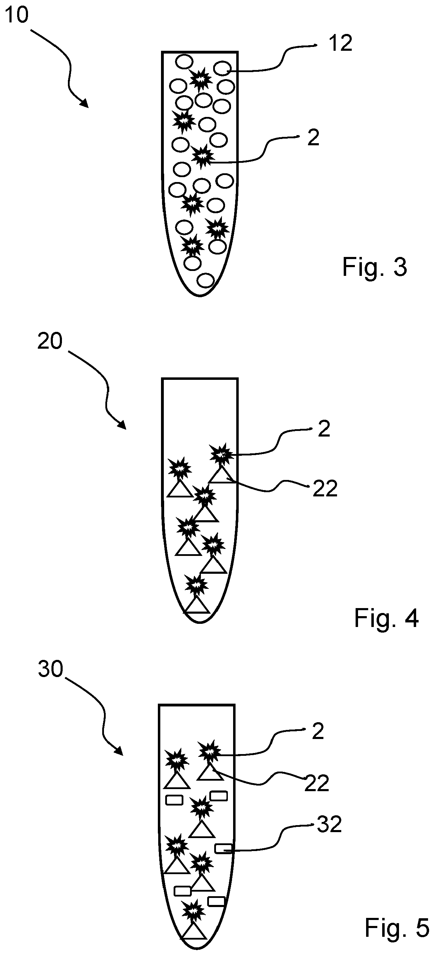

Die Figur 3 zeigt eine Testprobe 10, welche den zu detektierenden Erreger 2 und Körperflüssigkeiten 12 aufweist. Andere Testproben 10 können auch keine zu detektierenden Erreger 2 aufweisen. Dies ist davon abhängig ob der Proband den Erreger 2 in sich trägt oder nicht. Je nach Art der Testproben-Entnahme können zusätzlich oder alternativ zu den Körperflüssigkeiten 12 auch feste Bestandteile in der Testprobe 10 vorhanden sein.

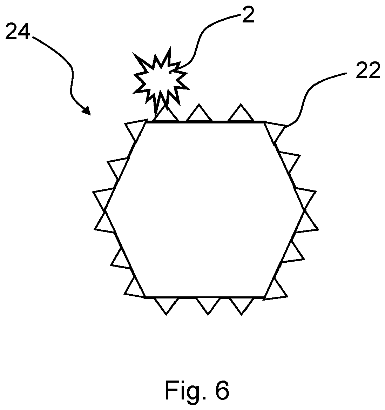

Figur 4 zeigt eine aufgereinigte Testprobe 20 mit selektiven Rezeptoren 22 und zu detektierende Erreger 2. Andere aufgereinigte Testroben 20 können keine zu detektierenden Erreger 2 aufweisen. Dies ist davon abhängig ob die korrespondierende Testprobe 10 die zu detektierenden Erreger 2 aufweist. In einem alternativen nicht dargestellten Ausführungsbeispiel kann die aufgereinigte Testprobe 20 auch mit den selektiven Rezeptoren 22 bestückte Mikropartikel (Figur 6), insbesondere magnetische Mikropartikel 24 aufweisen. Die magnetischen Mikropartikel 24 weisen einen von Kieselgel ummantelten Eisenoxidkern auf. An der Oberfläche der magnetischen Mikropartikel 24 sind die selektiven Rezeptoren 22 angeordnet. Insbesondere können die magnetischen Mikropartikel 24 ein paramagnetisches Material aufweisen, so dass sie in ein externes Magnetfeld hineingezogen werden können.

Figur 5 zeigt eine Messprobe 30 mit selektiven Rezeptoren 22, zu detektierenden Erregern 2 und einer Flüssigkeit 32. Die Flüssigkeit 32 kann als Pufferlösung wirken.

Andere aufgereinigte Testroben 20 können keine zu detektierenden Erreger 2 und/oder keine Flüssigkeit 32 aufweisen. Dies ist davon abhängig ob die korrespondierende Testprobe 10 bzw. die korrespondierende aufgereinigte Testprobe 20 die zu detektierenden Erreger 2 aufweist und ob der korrespondierenden aufgereinigten Testprobe 20 Flüssigkeit 32 hinzugefügt wurde.

In einem alternativen nicht dargestellten Ausführungsbeispiel kann die Messprobe 30 auch mit den selektiven Rezeptoren 22 bestückte Mikropartikel (Figur 6) aufweisen.

Wie aus den Figuren 1 und 2 ersichtlich ist, umfasst das Verfahren 1 zur Detektion von Erregern 2, insbesondere von Viren oder Virusgruppen oder Bakterien, folgende Verfahrensschritte.

Die entnommene Testprobe 10 wird aufgereinigt, insbesondere biochemisch aufgereinigt. Bei der Aufreinigung S200 werden in einem Verfahrensschritt S220 mehrere selektive Rezeptoren 22 der Testprobe 10 zugefügt, welche jeweils den vorhandenen zu detektierenden Erreger 2 binden. Das Binden der zu detektierenden Erreger 2 an die selektiven Rezeptoren 22 kann innerhalb von Zentelsekunden erfolgen. Hierbei können unter Umständen auch andere Erreger und/oder Proteine und/oder Moleküle an die selektiven Rezeptoren 22 gebunden werden, welche nicht den zu detektierenden Erregern 2 entsprechen.

In einem Trennverfahren S240 werden die selektiven Rezeptoren 22 mit den vorhanden an die selektiven Rezeptoren 22 gekoppelten zu detektierenden Erregern 2 von der Testprobe 10 getrennt.

Im dargestellten Ausführungsbeispiel werden bei der Aufreinigung S200, insbesondere der biochemischen Aufreinigung, magnetische Mikropartikel 24 mit selektiven Rezeptoren 22 im Verfahrensschritt S220 der Testprobe 10 zugeführt. In einem alternativen nicht dargestellten Ausführungsbeispiel können die selektiven Rezeptoren 22 durch andere geeignete Mikropartikel mit selektiven Rezeptoren 22 Testprobe 10 zugeführt werden.

Des Weiteren können die magnetischen Mikropartikel 24 zu einem anderen Zeitpunkt mit selektiven Rezeptoren 22 bestückt werden, so dass ein Bestücken der magnetischen Mikropartikel 24 mit selektiven Rezeptoren 22 keine Zeit im Trennverfahren S240 beansprucht. Zudem können anstelle der magnetischen Mikropartikel 24 auch nicht magnetische Mikropartikel 24 verwendet werden.

Im dargestellten Ausführungsbeispiel werden die magnetischen Mikropartikel 24 im Trennverfahren S240 in einem alternativen Verfahrensschritt S250 durch einen Magneten 312 festgehalten und vom Rest der Testprobe 10 getrennt. Hierbei wird der Rest der Testprobe 10 insbesondere verworfen.

Die festgehaltenen magnetischen Mikropartikel 24 bilden die aufgereinigte Testprobe 20. Die aufgereinigte Testprobe 20 umfasst hierbei die magnetischen Mikropartikel 24, die mit den magnetischen Mikropartikeln 24 gekoppelten selektiven Rezeptoren 22 und in der Testprobe 10 vorhandene Erreger 2 und/oder Moleküle und/oder Proteine, welche von den selektiven Rezeptoren 22 an die magnetischen Mikropartikel 24 gebunden sind. Durch ein anderes Trennverfahren S240 kann die aufgereinigte Testprobe 20 auch nur die selektiven Rezeptoren 22 und in der Testprobe 10 vorhandene Erreger 2 und/oder Moleküle und/oder Proteine, welche von den selektiven Rezeptoren 22 gebunden sind oder nicht magnetische Mikropartikel mit den entsprechenden selektiven Rezeptoren 22 und in der Testprobe 10 vorhandenen Erregern 2 und/oder Molekülen und/oder Proteine, aufweisen.

Der verworfene Rest der Testprobe 10 kann Körperflüssigkeiten 12 und/oder andere nicht zu detektierende Erreger und/oder Proteine und/oder Kohlenhydrate und/oder Moleküle umfassen, die für die Auswertung nicht von Interesse sind.

In einem alternativen nicht dargestellten Ausführungsbeispiel sind auch andere Trennverfahren S240 möglich. Beispielsweise können Mikropartikel mit selektiven Rezeptoren 22 bestückt werden, wobei diese Mikropartikel 24 mit den selektiven Rezeptoren 22 und den an die selektiven Rezeptoren 22 gebundenen Erregern 2 und/oder Proteinen und/oder Molekülen durch ein Sieb vom Rest der Testprobe 10 getrennt werden können.

Zum Gewinnen einer Messprobe 30 wird in Verfahrensschritt S260 die aufgereinigte Testprobe 20 mit einer Flüssigkeit 32 versetzt. Hierbei können die von der Testprobe 10 getrennten magnetischen Mikropartikel 24 zur Bildung der Messprobe 30 in einer Flüssigkeit 32 suspendiert werden. Als Flüssigkeit 32 kann Deuteriumoxid verwendet werden. Es sind auch andere Flüssigkeiten 32 vorstellbar, insbesondere Flüssigkeiten 32, welche ein optisches Spektrum aufweisen, welches ein optisches Spektrum der zu detektierenden Erreger 2 nicht überlagert. Des Weiteren ist das Zugeben der Flüssigkeit 32 auch dann möglich, wenn die aufgereinigte Testprobe 20 keine magnetischen Mikropartikel 24 aufweist. Des Weiteren kann auf die Zugabe von Flüssigkeit 32 auch verzichtet werden.

In einem zusätzlichen nicht dargestellten Verfahrensschritt können die Mikropartikel 24, falls Mikropartikel 24 im Trennverfahren S240 der Testprobe 10 zugefügt wurden, von den zu detektierenden Erregern 2 getrennt werden. Dadurch umfasst die Messprobe 30 keine Mikropartikel 24 und lediglich selektive Rezeptoren 22, an welche die entsprechenden Erreger 2 und/oder Proteine und/oder Moleküle gebunden sind. Zusätzlich oder alternativ könnten auch die Erreger von den selektiven Rezeptoren 22 getrennt werden.

Im dargestellten Ausführungsbeispiel weist die aus der Aufreinigung S200, insbesondere aus der biochemischen Aufreinigung, gewonnene Messprobe 30 die selektiven Rezeptoren 22 mit an selektiven Rezeptoren 22 gebundenen, zu detektierende Erreger 2 und die Flüssigkeit 32 auf. Sind in der Testprobe 10 keine zu detektierenden Erreger 2 vorhanden, umfasst die korrespondierende Messprobe 30 auch keine zu detektierenden Erreger 2. Des Weiteren kann die Messprobe 30 leichte nicht dargestellte Verunreinigungen und/oder Mikropartikel 24 aufweisen.

Die in Figur 1 dargestellten Verfahrensschritte S200 bis S260 können in wenigen Minuten, beispielsweise ein bis fünf Minuten, durchgeführt werden. Des Weiteren können mehrere Testproben 10 zeitgleich, insbesondere parallel, chemisch aufgereinigt werden.

In einem optischen Verfahren S300 durch eine laserbasierte Infrarot-Spektroskopie S320 werden die in der Messprobe 30 vorhandenen zu detektierenden Erreger 2 nachgewiesen. Hierbei wird im dargestellten Ausführungsbeispiel in einem Verfahrensschritt S340 ein optisches Messspektrum 40 der Messprobe 30 aufgenommen. In einem Verfahrensschritt S380 wird das aufgenommene optische Messspektrum 40 mit wenigstens einem Referenzspektrum 42 verglichen. Alternativ zur laserbasierten Infrarot-Spektroskopie S320 sind auch andere geeignete optische Verfahren zum Nachweisen vorhandener zu detektierenden Erreger 2 vorstellbar.

In einem nicht dargestellten Verfahrensschritt wird die Messprobe 300 in einer Messzelle 430 angeordnet und die Messzelle 430 wird von einer geeigneten Quelle bestrahlt. Ein Sensor 260 erfasst die reflektierte oder transmittierte Strahlung und nimmt so das Messspektrum 40 auf. Das Aufnehmen des Messspektrums 40 kann innerhalb von 10 s erfolgen.

Im dargestellten Ausführungsbeispiel des Verfahrens 1 wird zur Erfassung des optischen Messspektrums 40 ein durchstimmbarer Laser 410, insbesondere ein Quantenkaskadenlaser 412, verwendet. Es sind auch andere Quellen insbesondere Infrarot-Quellen beispielsweise Globarstrahler oder andere geeignete Laser vorstellbar. Ein Globarstrahler kann ein elektrisch auf 980 bis 1650 Grad Celsius erhitzter Siliziumcarbid-Stab sein, welcher Strahlung im mittleren und fernen Infrarotbereich homogen emittiert.

Im dargestellten Ausführungsbeispiel des Verfahrens 1 wird im Verfahrensschritt S360 wenigstens ein Referenzspektrum 42 zeitgleich mit dem Messspektrum 40 aufgenommen. Die Referenzprobe 50 ist in einer baugleichen Messzelle 430 angeordnet und umfasst ebenfalls die Flüssigkeit 32, die selektiven Rezeptoren 22 und gegebenenfalls die magnetischen Mikropartikel 24, welche auch die Messprobe 30 umfasst.

Durch diese Referenzmessung kann beispielsweise ein Differenzspektrum aus Referenzspektrum 42 und Messspektrum 40 gebildet werden, welches von der Flüssigkeit 32 oder von den magnetischen Mikropartikel 24 oder von Messzelle 430 stammende Signale nicht mehr oder nur im geringen Maße aufweist. Dadurch können von den zu detektierenden Erregern 2 stammende Signale deutlich im Differenzspektrum hervortreten. Dadurch kann die Komplexität der Messspektren 42 verringert und die Identifikation der zu detektierenden Erreger 2 erleichtert werden.

Zusätzlich oder alternativ kann in einem zusätzlichen Verfahrensschritt wenigstens ein Referenzspektrum 42 aufgenommen und hinterlegt werden. Hierbei kann die Referenzprobe 50 zumindest den zu detektierenden Erreger 2 aufweisen. Dadurch kann ein Fingerabdruck des zu detektierenden Erregers 2 erstellt und hinterlegt werden. Die Messspektren 40 können auf das vorhanden sein dieses Fingerabdrucks überprüft werden. Alternativ oder zusätzlich kann die Referenzprobe 50 den zu detektierenden Erreger 2 nicht aufweisen. Die Messspektren 40 können mit diesem Referenzspektrum auf zusätzliche Signale überprüft werden, welche auf das Vorhandensein anderer Elemente in der Messprobe 30 als in der Referenzprobe 50 hinweisen. Es besteht zudem die Möglichkeit aufgenommene Messspektren 40 als Referenzspektren 42 zu hinterlegen.

In einem weiteren nicht dargestellten Verfahrensschritt werden bei Entdeckung neuer Erregermutationen die selektiven Rezeptoren 22 angepasst. Des Weiteren können weitere Referenzspektren 42 aufgenommen und hinterlegt werden.

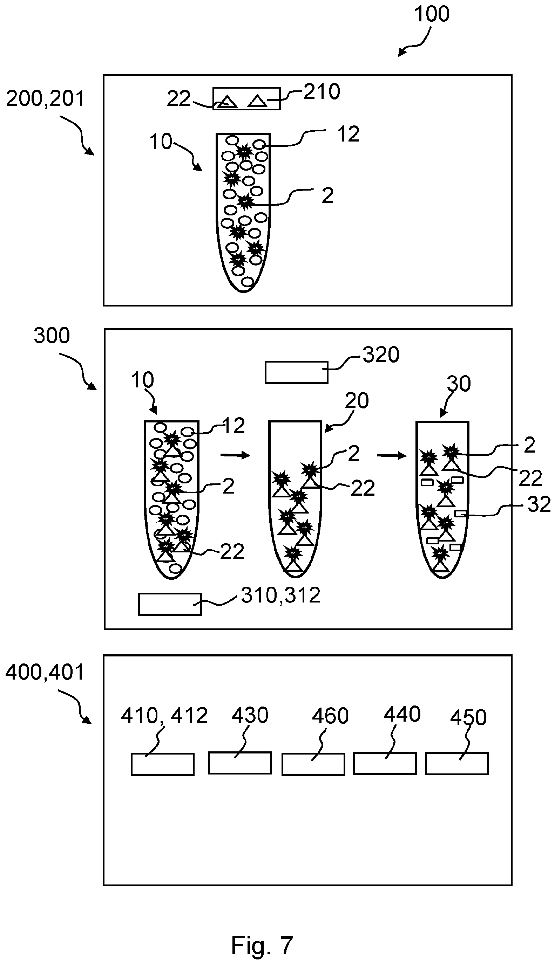

Die Figuren 7 bis 10 zeigen eine Analyseeinrichtung 100 oder Komponenten der Analyseeinrichtung 100, hergerichtet zur Durchführung eines Verfahrens zur Detektion von Erregern 2, insbesondere von Viren oder Virusgruppen oder Bakterien. Die Analyseeinrichtung 100 umfasst eine Vorbereitungsstation 200, eine Trennungsanordnung 300, und eine Auswertungsstation 400. Diese Komponenten müssen nicht am selben Ort angeordnet sein. Alternativ können die Komponenten zusammen in einem Gebäude oder einem Raum oder einem Bereich angeordnet sein.

Die Vorbereitungsstation 200 ist als Aufreinigungsstation 201, insbesondere als biochemische Aufreinigungsstation, ausgebildet, welche Mittel 210 zum Einfüllen von selektiven Rezeptoren 22 in eine Testprobe 10 umfasst, wobei die selektiven Rezeptoren 22 jeweils in der Testprobe 10 vorhandene zu detektierende Erreger 2 binden. Die Mittel 210 zum Einfüllen von selektiven Rezeptoren 22 können als Pipetten ausgeführt sein. Diese können matrixartig nebeneinander angeordnet sein. Mehrere Testproben 10 können in der gleichen Matrix angeordnet sein, so dass jeweils eine Pipette mit selektiven Rezeptoren 22 einer Testprobe 10 zugeordnet ist. Alternativ kann eine Pipette mit selektiven Rezeptoren 22 nacheinander den Testproben 10 die selektiven Rezeptoren 22 zuführen.

In einem Ausführungsbeispiel können die Mittel 210 zum Einfüllen von selektiven Rezeptoren 22 in eine Testprobe 10 magnetische Mikropartikel 24 mit selektiven Rezeptoren 22 der Testprobe 10 zuführen. Alternativ zu magnetischen Mikropartikeln 24 mit selektiven Rezeptoren 22 können die Mittel zum Einfüllen den Testproben 10 auch andere geeignete Mikropartikel mit selektiven Rezeptoren 22 zuführen.

Eine Trennungsanordnung 300 umfasst Mittel 310, welche die selektiven Rezeptoren 22 mit den an die selektiven Rezeptoren 22 gekoppelten Erreger 2 von der Testprobe 10 trennt und dadurch eine aufgereinigte Testprobe 20 gewinnt.

Die Trennungsanordnung 300 umfasst weitere Mittel 320, die von der Testprobe 10 getrennte selektive Rezeptoren 22 mit den gekoppelten Erregern 2 mit einer Flüssigkeit 32 versetzt und dadurch eine Messprobe 30 gewinnt. Auch die weiteren Mittel 320 können als Pipetten ausgeführt sein. Die Flüssigkeit 32 ist im dargestellten Ausführungsbeispiel Deuteriumoxid. Es sind aber auch andere geeignete Flüssigkeiten vorstellbar.

Im dargestellten Ausführungsbeispiel umfasst die Trennungsanordnung 300 Magneten 312, welche die magnetischen Mikropartikel 24 festhält und vom Rest der Testprobe 10 trennt, wobei die festgehalten Mikropartikel 24 eine aufgereinigte Testprobe 20 bilden. Mit den magnetischen Mikropartikel 24 werden auch die selektiven Rezeptoren 22 und die an die selektiven Rezeptoren 22 gebundenen Erreger und/oder Proteine und/oder Moleküle festgehalten.

Zum Trennen der magnetischen Mikropartikel 24 vom Rest der Testprobe 10 kann der Rest der Testprobe 10 beispielsweise abgesaugt oder ausgeschüttet werden, während die magnetischen Mikropartikel 24 von dem Magneten festgehalten werden. Es sind auch andere Trennungsanordnungen 300, beispielsweise Siebe, vorstellbar.

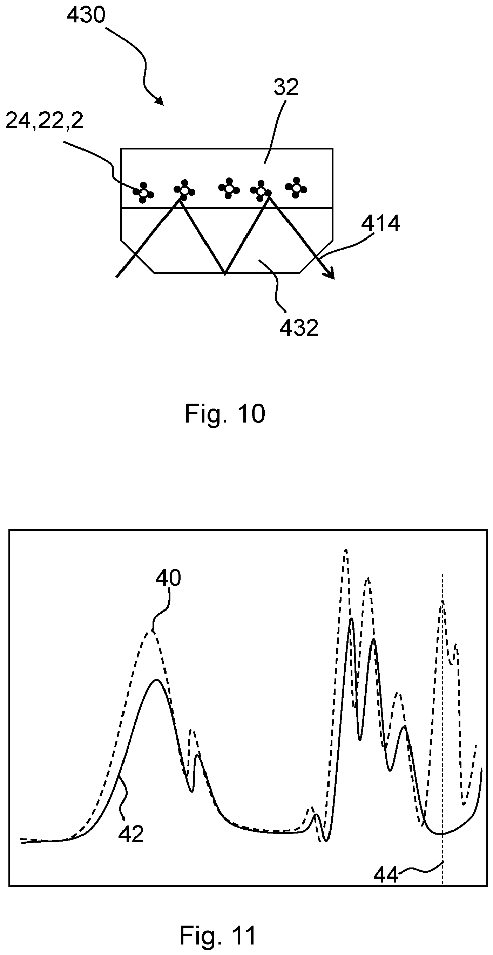

Die Auswertungsstation 400 ist als optische Auswertungsstation, insbesondere als eine laserbasierte Infrarot-Spektroskopieanordnung 401, ausgebildet. Die Auswertungsstation 400 nimmt wenigstens ein optisches Messspektrum 40 der Messprobe 30 auf und vergleicht das optische Messspektrum 40 mit wenigstens einem Referenzspektrum 42 (Figur 11). Ein Messspektrum 40 einer Messprobe 30 mit zu detektierenden Erregern 2 weist im Gegensatz zu einem Referenzspektrum 42 einer Referenzprobe 50 ohne zu detektierende Erreger 2 zusätzliche Peaks 44 auf, welche für den zu detektierenden Erreger 2 typisch sein können. Es können auch Referenzspektren 42 von Referenzproben 50 mit den zu detektierenden Erregern 2 hinterlegt sein. Dadurch können auch andere an selektiven Rezeptoren 22 gebundene Erreger und/oder Proteine und/oder Moleküle von den zu detektierenden Erregern 2 unterschieden werden. Es sind Referenzenspektren 42 von Referenzproben 50 mit diesen anderen Proteinen und/oder Molekülen und/oder Erregern für die Auswertung hinterlegbar.

Die Auswertungsstation 400 umfasst im dargestellten Ausführungsbeispiel zur Erfassung des optischen Messspektrums 40 einen durchstimmbaren Laser, insbesondere ein Quantenkaskadenlaser 412 und wenigstens einen Sensor 460. Der Quantenkaskadenlaser 412 wirkt als Quelle von Infrarotstrahlung im mittleren Infrarotstrahlungsbereich. Es sind auch andere Laser 210 und/oder Globarstrahler oder andere geeignete Quellen für Infrarotstrahlung vorstellbar.

Der Quantenkaskadenlaser 412 kann ein gepulster Laser mit einer Wiederholrate von 100 Hz bis 3 MHz und einer mittleren Leistung von 0.50 W sein. Der Quantenkaskadenlaser 412 scannt im Verlauf einer Messung den Wellenlängenbereich mit einer Geschwindigkeit von etwa 10 m/s ab. Die Dauer einer Messung liegt entsprechend unter einer Sekunde.

Der wenigstens ein Sensor 460 empfängt die transmittierte oder reflektierte Strahlung der Messzelle 430. Als Sensor 460 eignet sich im relevanten Spektralbereich ein peltiergekühlte Infrarot-Photodetektor mit Impedanzverstärker. Es sind auch andere Sensoren 460 vorstellbar.

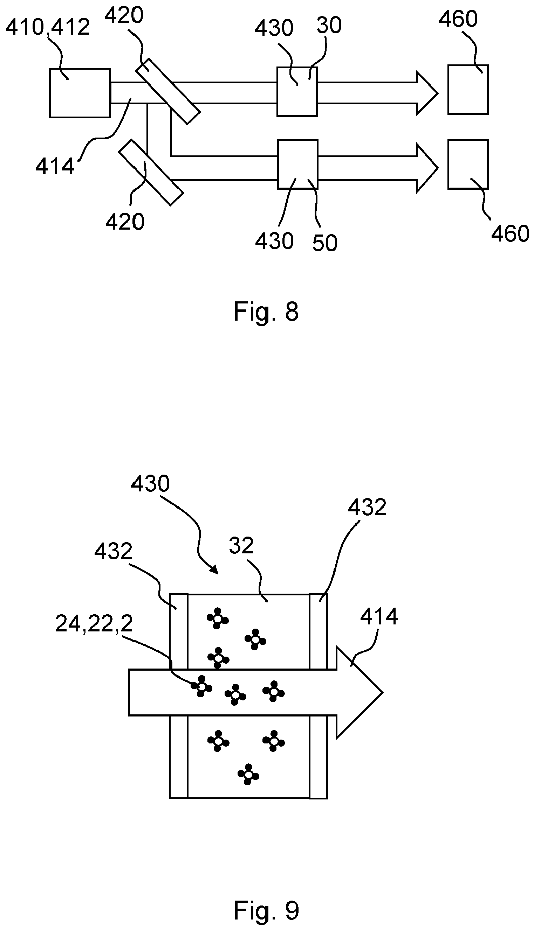

Figur 8 zeigt eine Detailansicht einer Auswertungsstation 400. Das dargestellte Ausführungsbeispiel der Auswertungsstation 400 kann wenigstens ein Referenzspektrum 42 zeitgleich mit dem Messspektrum 40 aufnehmen. Der Laserstrahl 414 des Quantenkaskadenlasers 412 wird an zwei Umlenkelementen 420 derart umgelenkt und geteilt, dass zwei Messzellen 430 bestrahlt werden. In Strahlungsrichtung ist hinter den jeweiligen Messzellen 430 ein Sensor 460 angeordnet, welcher die durch die entsprechende Messzelle 430 transmittierte Strahlung empfängt. Aus diesen Daten wird das Messspektrum 40 bzw. das Referenzspektrum 42 aufgezeichnet. Im dargestellten Ausführungsbeispiel sind die Messzellen 430, die korrespondierenden Laserstrahlen 414 und die Sensoren 460 parallel zueinander angeordnet. Es sind auch andere Anordnungen vorstellbar. Des Weiteren können in einem alternativen Ausführungsbeispiel die Sensoren 460 so zur Messzelle 430 angeordnet sein, dass die Sensoren 460 an der jeweiligen Messzelle 430 reflektierte Strahlungen empfangen.

In einer alternativen Ausgestaltung kann auch nur eine Messprobe 30 oder eine Referenzprobe 50 nacheinander untersucht werden. Alternativ können anstelle einer Messprobe 30 und einer Referenzprobe 50 auch zwei Messproben 30 oder zwei Referenzproben 50 zeitgleich untersucht werden. In einem weiteren Ausführungsbeispiel können auch mehr als zwei Proben 30, 50 gleichzeitig untersucht werden.

Die Figuren 9 und 10 zeigen Ausführungsbeispiele der Messzellen 430 der Auswertungsstation 400. In den dargestellten Ausführungsbeispielen sind die Messzellen 430 als Dünnschichtzellen mit einer möglichen Schichtdicke von 5 bis ca. 35 µm ausgebildet. Die Fenster 432 der Messzellen 430 transmittieren Infrarotstrahlung im mittleren Strahlungsbereich. Dadurch können die Laserstrahlen 414 in die Messzellen 430 eindringen. Die Fenster 432 können Kristallmaterial, beispielsweise Zinkselenid, Calciumfluorid oder Diamant, aufweisen.

Im in Figur 9 dargestellten Ausführungsbeispiel wird der Laserstrahl 414 durch die Messzelle 430 geführt und die transmittierte Strahlung gemessen.

Im in Figur 10 dargestellten Ausführungsbeispiel wird der Laserstrahl 414 an dem Fenster 432 und an der Messprobe 30 oder der Referenzprobe 50 reflektiert und die reflektierte Strahlung wird gemessen.

Die Auswertungsstation 400 kann wenigstens ein Referenzspektrum 42 aufnehmen und hinterlegen. Zur Auswertung können auch von anderen Auswertungsstationen 400 aufgenommene Referenzspektren 42 hinterlegt werden.

Die Auswertungsstation 400 umfasst wenigstens eine Auswerte- und Steuereinrichtung 440, welche das Messspektrum 40 auswertet und insbesondere entsprechende Signale an eine Ausgabeeinheit 450 ausgibt. Die Ausgabeeinheit 450 kann beispielsweise eine Alarmeinrichtung, beispielsweise eine Warn-App, des korrespondierenden Probanden sein. Es sind auch andere geeignete Ausgabeeinheiten 450 vorstellbar. Die Ausgabeeinheit 450 kann insbesondere die Information, ob das Testergebnis positiv oder negativ ist, ausgeben. Zusätzlich kann die Ausgabeeinheit 450 über die Art des Erregers beispielsweise über eine entdeckte Mutation Informieren.

Eine nicht dargestellte Verwendung einer solchen Analyseeinrichtung 100 ist dem korrespondierenden Verfahrens 1 (Figuren 1 und 2) zur Detektion von Erregern 2, insbesondere von Viren oder Virusgruppen oder Bakterien sehr ähnlich. Der Hauptunterschied besteht im Wesentlichen darin, dass sich die Verwendungsschritte explizit auf die Analyseeinrichtung 100 bzw. auf Komponenten der Analyseeinrichtung 100 beziehen, während die Verfahrensschritte des Verfahrens 1 auch mit andren geeigneten Analyseeinrichtungen durchgeführt werden können.

Bei der Verwendung der Analyseeinrichtung 100 wird in einer Vorbereitungsstation 200 eine entnommene Testprobe 10 aufgereinigt, insbesondere biochemisch aufgereinigt. Bei der Aufreinigung S200 werden der Testprobe 10 Mikropartikel 24 mit einer funktionalisierten Oberfläche und mit wenigstens einem selektiven Rezeptor 22 zugeführt welche jeweils den vorhandenen zu detektierenden Erreger 2 binden. In einer Trennungsanordnung 300 werden in einem Trennverfahren S240 die selektiven Rezeptoren 22 mit den vorhanden an die selektiven Rezeptoren 22 gekoppelten zu detektierenden Erregern 2 von der Testprobe 10 getrennt.

Zum Gewinnen einer Messprobe 30 aus einer aufgereinigten Testprobe 20, welche von der Testprobe 10 getrennte selektive Rezeptoren 22 und vorhandene an die selektiven Rezeptoren 22 gekoppelte zu detektierende Erreger 2 umfasst, wird die aufgereinigte Testprobe 20 mit einer Flüssigkeit 32 versetzt. In einer Auswertungsstation 400 werden in einem optischen Verfahren S300, insbesondere durch eine laserbasierte Infrarot-Spektroskopie S320, die in der Messprobe 30 vorhandenen zu detektierenden Erreger 2 nachgewiesen.

Weitere alternative oder zusätzliche Ausgestaltungen des beschriebenen Verfahrens 2 sind auch auf die Verwendung der Analyseeinrichtung 100 anwendbar.

In einer nicht dargestellten Verwendung eines selbstlernenden Netzwerks zur Auswertung von optischen Messspektren 40 von Messproben 30, welche zum Nachweis von Erregern 2 entnommen werden, werden mehrere Messspektren 40 mit und/oder ohne bekannte Erregerlast zur Auswertung hinterlegt. Die Auswertung der Messspektren 40 wird fortlaufend angepasst. Das selbstlernende Netzwerk kann neue Mutationen des Erregers erkennen. Hierbei kann das selbstlernende Netzwerk eine Warnung an eine Ausgabeeinheit 450 ausgegeben.

### Bezugszeichen

- 1: Verfahren zur Detektion eines Erregers
- 2: Erreger
- 10: Testprobe
- 12: Körperflüssigkeit
- 20: aufgereinigte Testprobe
- 22: selektive Rezeptoren
- 24: magnetische Mikropartikel
- 30: Messprobe
- 32: Flüssigkeit
- 40: Messspektrum
- 42: Referenzspektrum
- 44: virusbedingter Peak
- 50: Referenzprobe
- 100: Analyseeinrichtung
- 200: Vorbereitungsstation
- 201: Aufreinigungsstation
- 210: Mittel zum Einfüllen von selektiven Rezeptoren
- 300: Trennanordnung
- 310: Mittel zur Trennung
- 312: Magnet
- 320: Mittel zum Versetzten mit Flüssigkeit
- 400: Auswertungsstation
- 401: Infrarot-Spektroskopieanordnung
- 410: Laser
- 412: Quantenkaskadenlaser
- 414: Laserstrahl
- 420: Umlenkelemente
- 430: Messzelle
- 432: Fenster
- 440: Auswerte und Steuereinrichtung
- 450: Ausgabeeinheit
- 460: Sensor
- S200: Aufreinigung
- S220: hinzugeben von selektiven Rezeptoren
- S240: Trennverfahren
- S250: Festhalten magnetic Beats durch Magneten
- S260: versetzten/suspendieren mit einer Flüssigkeit
- S300: Verfahrensschritt optisches Verfahren
- S320: Infrarot-(Schwingungs)spektroskopie
- S340: Aufnehmen eines Messspektrums
- S360: Aufnehmen eines Referenzspektrums
- S380: Vergleichen von Messspektrum und Referenzspektrum

## Patentansprüche

1. Verfahren (1) zur Detektion von Erregern (2), insbesondere von Viren oder Virusgruppen oder Bakterien,
wobei eine entnommene Testprobe (10) aufgereinigt wird,
wobei der Testprobe (10) bei der Aufreinigung (S200) Mikropartikel (24) mit einer funktionalisierten Oberfläche und mit wenigstens einem selektiven Rezeptor (22) zugeführt werden, welcher den vorhandenen zu detektierenden Erreger (2) bindet,
wobei in einem Trennverfahren (S240) der wenigstens eine selektive Rezeptor (22) mit den vorhanden an den wenigstens einen selektiven Rezeptor (22) gekoppelten zu detektierenden Erregern (2) von der Testprobe (10) getrennt werden,
wobei als Messprobe (30) eine aufgereinigte Testprobe (20) verwendet wird, welche den wenigstens einen von der Testprobe (10) getrennten selektiven Rezeptor (22) und vorhandene an den wenigstens einen selektiven Rezeptor (22) gekoppelte Erreger (2) umfasst, wobei in einem optischen Verfahren (S300) in Form von laserbasierter Infrarot-Spektroskopie (S320) die in der Messprobe (30) vorhandenen, zu detektierenden Erreger (2) nachgewiesen werden.

2. Verfahren nach Anspruch 1, wobei zum Gewinnen einer Messprobe (30) die aufgereinigte Testprobe (20), welche den wenigstens einen von der Testprobe (10) getrennten selektiven Rezeptor (22) und vorhandene an den wenigstens einen selektiven Rezeptor (22) gekoppelte Erreger (2) umfasst, mit einer Flüssigkeit (32) versetzt wird,
insbesondere, wobei als Flüssigkeit (32) Deuteriumoxid verwendet wird.

3. Verfahren nach Anspruch 1 oder 2, wobei bei der Aufreinigung (S200) magnetische Mikropartikel (24) mit einer funktionalisierten Oberfläche und mit wenigstens einem selektiven Rezeptor (22) der Testprobe (10) zugeführt werden, insbesondere wobei die magnetischen Mikropartikel (24) durch einen Magneten (312) fixierbar oder durch einen Magneten (312) in eine vorgegebene Richtung bewegbar sind.

4. Verfahren nach einem der vorhergehenden Ansprüche, wobei in einem Trennverfahren (S240) die Mikropartikel (24) vom Rest der Testprobe (10) getrennt werden, insbesondere wobei die abgetrennten Mikropartikel (24) die aufgereinigte Testprobe (20) bilden.

5. Verfahren nach einem der vorhergehenden Ansprüche, wobei die von der Testprobe (10) getrennten Mikropartikel (24) zur Bildung der Messprobe (30) in einer Flüssigkeit (32) suspendiert werden.

6. Verfahren nach einem der vorhergehenden Ansprüche, wobei mehrere Testproben (10) zeitgleich parallel chemisch oder biochemisch aufgereinigt werden, und/oder wobei mehrere Testproben (10) in einer Vorbereitungsstation (200) und in einer Trennungsanordnung (300) parallel bearbeitet werden.

7. Verfahren nach einem der vorhergehenden Ansprüche, wobei wenigstens ein Referenzspektrum (42) zeitgleich mit einem Messspektrum (40) aufgenommen wird, und/oder
wobei wenigstens ein Referenzspektrum (42) aufgenommen und hinterlegt wird.

8. Verfahren nach einem der vorhergehenden Ansprüche, wobei bei Entdeckung neuer Erregermutationen die selektiven Rezeptoren (22) angepasst werden.

9. Analyseeinrichtung (100), hergerichtet zur Durchführung eines Verfahrens zur Detektion von Erregern (2), insbesondere von Viren oder Virusgruppen oder Bakterien, welche eine Vorbereitungsstation (200), eine Trennungsanordnung (300), und eine Auswertungsstation (400) umfasst,
wobei die Vorbereitungsstation (200) als Aufreinigungsstation (201) ausgebildet ist, welche Mittel (210) zum Einfüllen von wenigstens einem selektiven Rezeptor (22) in eine Testprobe (10) umfasst, wobei die Mittel (210) zum Einfüllen von selektiven Rezeptoren (22) Mikropartikel (24) mit einer funktionalisierten Oberfläche und mit wenigstens einem selektiven Rezeptor (22) der Testprobe (10) zuführen,
wobei der wenigstens eine selektive Rezeptor in der Testprobe (10) vorhandene zu detektierende Erreger (2) bindet, und
wobei eine Trennungsanordnung (300) Mittel (310) umfasst, welche den wenigstens einen selektiven Rezeptor (22) mit den an den wenigstens einen selektiven Rezeptor (22) gekoppelten Erreger (2) von der Testprobe (10) trennt und dadurch eine aufgereinigte Testprobe (20) gewinnt, welche einer Messprobe (30) entspricht,
wobei die Auswertungsstation (400) als optische Auswertungsstation, als eine laserbasierte Infrarot-Spektroskopieanordnung (401), ausgebildet ist, welche wenigstens ein optisches Messspektrum (40) der Messprobe (30) aufnimmt und mit wenigstens einem Referenzspektrum (42) vergleicht.

10. Analyseeinrichtung nach Anspruch 9, wobei die Trennungsanordnung (300) weitere Mittel (320) umfasst, welche die aufgereinigte Testprobe (20) mit dem wenigstens einen von der Testprobe (10) getrennten selektiven Rezeptor (22) mit vorhandenen an den wenigstens einen selektiven Rezeptor (22) gekoppelten Erregern (2) mit einer Flüssigkeit (32) versetzt, insbesondere Deuteriumoxid, und dadurch eine Messprobe (30) gewinnt.

11. Analyseeinrichtung nach Anspruch 9 oder 10, wobei die Mittel (210) zum Einfüllen von selektiven Rezeptoren (22) in eine Testprobe (10) magnetische Mikropartikel (24) mit einer funktionalisierten Oberfläche und mit wenigstens einem selektiven Rezeptor (22), der Testprobe (10) zuführen.

12. Analyseeinrichtung nacheinem der Ansprüche 9 bis 11, wobei die Trennungsanordnung (300) Mittel (312) umfasst, welche Mikropartikel (24) festhält und vom Rest der Testprobe (10) trennt, wobei die festgehalten Mikropartikel (24) eine aufgereinigte Testprobe (20) bilden.

13. Analyseeinrichtung nach einem der Ansprüche 9 bis 12, wobei die Auswertungsstation (400) zur Erfassung des optischen Messspektrums (40) einen durchstimmbaren Laser, insbesondere ein Quantenkaskadenlaser (412) und wenigstens einen Sensor (460), umfasst.

14. Analyseeinrichtung nach einem der Ansprüche 9 bis 13, wobei die Auswertungsstation (400) wenigstens ein Referenzspektrum (42) zeitgleich mit dem Messspektrum (40) aufnimmt, und/oder
wobei die Auswertungsstation (400) wenigstens ein Referenzspektrum (42) aufnimmt und hinterlegt.

15. Analyseeinrichtung nach einem der Ansprüche 9 bis 14, wobei die Auswertungsstation (400) wenigstens eine Auswerte- und Steuereinheit (440) umfasst, welche das Messspektrum (40) auswertet und insbesondere entsprechende Signale an eine Ausgabeeinheit (450) ausgibt.

16. Verwendung einer Analyseeinrichtung (100) nach einem der Ansprüche 9 bis 15,
wobei in einer Vorbereitungsstation (200) eine entnommene Testprobe (10) aufgereinigt wird,
wobei bei der Aufreinigung (S200) der Testprobe (10) Mikropartikel (24) mit einer funktionalisierten Oberfläche und mit wenigstens einem selektiven Rezeptor (22) zugeführt werden, welcher den vorhandenen zu detektierenden Erreger (2) bindet,
wobei in einer Trennungsanordnung (300) in einem Trennverfahren (S240) der wenigstens eine selektive Rezeptor (22) mit den vorhanden an den wenigstens einen selektiven Rezeptor (22) gekoppelten zu detektierenden Erreger (2) von der Testprobe (10) getrennt werden,
wobei eine Messprobe (30) einer aufgereinigten Testprobe (20) entspricht, welche den wenigstens einen von der Testprobe (10) getrennten selektiven Rezeptor (22) und vorhandene an den wenigstens einen selektiven Rezeptor (22) gekoppelte zu detektierende Erreger (2) umfasst,
wobei in einer Auswertungsstation (400) in einem optischen Verfahren (S300), durch eine laserbasierte Infrarot-Spektroskopie (S320), die in der Messprobe (30) vorhandenen zu detektierenden Erreger (2), nachgewiesen werden,
insbesondere wobei zum Gewinnen einer Messprobe (30) aus einer aufgereinigten Testprobe (20), welche den wenigstens einen von der Testprobe (10) getrennten selektiven Rezeptor (22) und vorhandene an den wenigstens einen selektiven Rezeptor (22) gekoppelte zu detektierende Erreger (2) umfasst, die aufgereinigte Testprobe (20), mit einer Flüssigkeit (32) versetzt wird.

17. Verwendung eines selbstlernenden Netzwerks zur Auswertung von optischen Messspektren (40) von Messproben (30), welche zum Nachweis von Erregern entnommen werden, wobei mehrere Messspektren (40) mit und/oder ohne bekannte Erregerlast zur Auswertung hinterlegt werden und die Auswertung fortlaufend angepasst wird.

18. Verwendung nach Anspruch 17, wobei neue Mutationen des Erregers (2) erkannt werden, insbesondere wobei bei Erkennen einer neuen Mutation des Erregers (2) eine Warnung an eine Ausgabeeinheit (450) ausgegeben wird.
